(19) Europäisches Patentamt / European Patent Office / Office européen des brevets

(11) **EP 4 668 278 A1**

(12) **EUROPEAN PATENT APPLICATION**

(43) Date of publication:
24.12.2025 Bulletin 2025/52

(21) Application number: 24305992.0

(22) Date of filing: 21.06.2024

(51) International Patent Classification (IPC):
*G16B 25/10* (2019.01)        *G16B 40/20* (2019.01)

(52) Cooperative Patent Classification (CPC):
**G16B 25/10; G16B 40/20**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **SANOFI**
75008 Paris (FR)

(72) Inventor: **NOURI, Nima**
**Cambridge, 02141 (US)**

(74) Representative: **Trichard, Louis et al**
**Venner Shipley LLP**
**200 Aldersgate**
**London EC1A 4HD (GB)**

(54) **METHOD, SYSTEM AND APPARATUS FOR GENERATING AND/OR AUGMENTING GENE EXPRESSION PROFILE DATASETS**

(57)      Methods, systems and apparatus are described for generating synthetic gene expression profile datasets for downstream biological/pharmacological analysis, processing and/or applications. Generating synthetic gene expression profile datasets includes: receiving a gene expression profile dataset comprising data representative of at least one gene expression profile; computing a complex gene expression profile dataset based on applying a discrete Fourier transform to data representative of each gene expression profile of the received gene expression profile dataset; sampling the complex gene expression profile dataset based on using a statistical distribution to sample and modify a predetermined number of one or more components of each complex gene expression profile for generating a plurality of synthetic complex gene expression profiles; computing a real-valued synthetic gene expression profile dataset based on applying an inverse discrete Fourier transform to the synthetic complex gene expression profile dataset; and outputting data representative of the real-valued synthetic gene expression profile dataset for downstream biological/pharmacological analysis, processing and/or applications.

FIG. 1A

EP 4 668 278 A1

**Description**

**Field**

This specification relates to methods, systems, and apparatus for generating and augmenting gene expression profile datasets from real-world gene expression profile datasets for use in downstream analysis / detection including *in silica*, *in vitro* and/or *in vivo* analysis and drug discovery and optimization.

**Background**

Gene expression profile datasets have been used in biology research for more than 20 years. They are now widely and routinely used for many different technical applications, including for example disease stratification, diagnosis, and prognosis, understanding the fundamental biology of disease processes, analyzing, and predicting responses to therapeutics and their mechanisms of action, analyzing tissue and cell variability and biology, and endotype, phenotype and biomarker identification. The application of gene expression profiles such as, without limitation, for example single-cell RNA sequencing (scRNA-seq) has become increasingly prominent in industrial settings, notably for its pivotal role in elucidating disease mechanisms and treatment responses at the cellular level. Simultaneously, advancements in deep learning and artificial intelligence (AI) or machine learning (ML) are revolutionizing medicinal and bioinformatics applications, particularly in drug discovery. The burgeoning integration of AI/ML into single-cell discovery has intensified the demand for extensive gene expression profile training datasets for training respective AI/ML models for applications such as, without limitation, for example drug discovery and/or diagnosing disease. For these technologies to perform efficiently, their AI/ML models must be trained on a comprehensive ensemble of high-quality training gene expression datasets or fine-tuned on a set of domain-specific biological data. However, economic and ethical considerations, along with the scarcity of available patient samples, often hinder AI/ML models and their application. This limitation is particularly acute in clinical settings involving rare diseases, where tasks like patient stratification and endotyping are critical. Hence, the inevitable need for extensive gene expression profile datasets significantly burdens research institutions, underscoring the urgency for cost-efficient and time-conscious solutions.

In response, *in silica* data generation strategies for generating gene expression profile training datasets are primarily oriented towards global pattern-learning techniques based on: (1) manifold-based and (2) neural network-based approaches. Among the manifold-based approaches are geometry and density-based methods. These methods rely on the presumed existence and precise identification of low-dimensional manifolds within, for example, gene expression profile or scRNA-seq datasets. However, their reliance on dimensionality reduction strategies inevitably comes at the cost of neglecting the nuances of individual cells, thus making them vulnerable to overlooking variations present in high-dimensional data. Moreover, the performance of these methods also depends on the presence of a substantial number of cells, which is necessary to explore the corresponding embedded manifolds effectively. More recently, generative models such as Generative Adversarial Networks (GANs) and Variational Autoencoders (VAEs) from within the AI/ML sphere have ventured to alleviate the data scarcity challenge present in transcriptomic studies. However, despite these efforts, they too inadvertently enter a paradoxical cycle: their performance remains challenged by the same limited sample size availability they seek to overcome. Furthermore, these types of models are notoriously time-consuming, resource-intensive, and heavily reliant on data-specific tuning of hyperparameters, ranging from architectural features such as the number of layers, layer width, and latent space dimensionality to training configurations, including learning rates, loss functions, and numbers of epochs. None of these techniques are suitable for reliably synthesizing gene expression profiles associated with a limited real-world gene expression profile dataset and, so, inadvertently perpetuate a cycle of data inadequacy.

**Summary**

According to a first aspect, there is provided a computer-implemented method for generating synthetic gene expression profile datasets for downstream biological/pharmacological analysis, processing and/or applications, the method comprising: receiving a gene expression profile dataset comprising data representative of at least one gene expression profile; computing a complex gene expression profile dataset based on applying a discrete Fourier transform to data representative of each gene expression profile of the received gene expression profile dataset; sampling the complex gene expression profile dataset based on using a statistical distribution to sample and modify a predetermined number of one or more components of each complex gene expression profile for generating a plurality of synthetic complex gene expression profiles; computing a real-valued synthetic gene expression profile dataset based on applying an inverse discrete Fourier transform to each of the plurality of synthetic complex gene expression profiles; and outputting data representative of the real-valued synthetic gene expression profile dataset for downstream biological/pharmacological analysis, processing and/or applications.

The computer-implemented method of the first aspect, wherein receiving the gene expression profile dataset further comprises receiving a gene expression profile dataset derived from real-world measurements of gene expression from one or more cells of at least one subject.

The computer-implemented method of the first aspect, wherein the downstream biological/pharmacological analysis, processing and/or applications comprise one or more from the group of: drug discovery or optimization; therapeutics; novel gene discovery; patient stratification; endotyping; disease detection/prevention; *in vivo* trials, *in vitro* wet lab analysis, and/or high-throughput screening laboratory analysis; any other type of biological/pharmacological analysis, processing and/or applications using at least the output real-valued synthetic gene expression profile dataset and, optionally, the corresponding real-world gene expression profile dataset; any other type of technical biological/-pharmacological processing or applications using at least the output real-valued synthetic gene expression profile dataset and, optionally, the corresponding real-world gene expression profile dataset.

The computer-implemented method of the first aspect, wherein: each gene expression profile is represented as a vector of length $N$, where $N > 0$ is the total number of genes and each element, n, of the vector represents a gene index of the gene expression profile for $0 \leq n \leq (N-1)$; and each complex gene expression profile is represented as a vector of length $N$, where $N$ is the total number of genes and each $k$-th element of the vector represents a complex component comprising a complex value with an amplitude and phase, for $0 \leq k \leq (N-1)$.

The computer-implemented method of the first aspect, wherein the gene expression profile dataset comprises data representative of gene expression profiles based on one from the group of: cell gene expression profiles; single-cell Ribonucleic acid, RNA, sequences; or any other particular type of gene expression profile associated with a subject or cellular structure.

The computer-implemented method of the first aspect, wherein said sampling the complex gene expression profile dataset for generating a plurality of synthetic complex gene expression profiles further comprising: computing the mean and variance for each complex component of each complex gene expression profile of the complex gene expression profile dataset; selecting a statistical distribution having said mean and variance for each complex component of the complex gene expression profile dataset; iteratively performing, until a predetermined number of synthetic complex gene expression profiles have been generated, the following steps of: generating, by sampling the statistical distribution for each complex component, sampled modification factors corresponding to the $N$ complex components of each complex gene expression profile; generating synthetic complex gene expression profiles based on applying, to each complex gene expression profile, a predetermined number of randomly selected sampled modification factors to corresponding complex components and their conjugate pairs of said each complex gene expression profile; and updating the synthetic complex gene expression profile dataset with the generated synthetic complex gene expression profiles.

The computer-implemented method of the first aspect, wherein: when the length of the gene expression profile, $N$, is an even number, then the complex conjugate pair of the (N/2)-th complex component is itself and the remaining complex conjugate pair of a $k$-th complex component, for $1 \leq k < N/2$, is the *(N-k)-th* complex component; and when the length of the gene expression profile, $N$, is an odd number, then the complex conjugate pair of a $k$-th complex component, for $1 \leq k < (N-1)/2$, is the *(N-k)-th* complex component.

The computer-implemented method of the first aspect, wherein the component of each complex gene expression profile corresponding to the Direct Current component is excluded from being randomly selected when applying sample modification factors.

The computer-implemented method of the first aspect, further comprising: determining one or more cell groups in the received gene expression profile of the complex gene expression profile dataset in which each cell group has a similar or the same gene expression profile; and wherein said sampling the generated complex gene expression profiles for generating a plurality of synthetic complex gene expression profiles is performed independently for each determined one or more cell groups.

The computer-implemented method of the first aspect, wherein each cell group is associated with the same cell-type of the same cluster of cells.

The computer-implemented method of the first aspect, wherein: when the number of cells in each cell group is greater than 1, then the selected statistical distribution used to generate each sample modification factor of each complex component uses a mean of 2 and the computed variance for said complex component for that cell group; and when the number of cells in each cell group is 1, then the selected statistical distribution used to generate each sample modification factor of each complex component uses a mean of 2 and a variance of 1.

The computer-implemented method of the first aspect, wherein the statistical distribution comprises a probability distribution based on a Gaussian or Normal distribution.

The computer-implemented method of the first aspect, wherein the predetermined number of one or more complex components is selected by a user, wherein the variability of the resulting synthetic gene expression profiles in relation to corresponding real-world gene expression profiles increases as the predetermined number of components increases.

The computer-implemented method of any preceding claim, wherein said outputting data representative of the real-value synthetic gene expression profile dataset for downstream biological/pharmacological analysis, processing and/or applications further comprises: generating an augmented gene expression profile dataset based on the real-value synthetic gene expression profile dataset and the real-world gene expression profile dataset; applying the augmented gene expression profile dataset to a foundational model for training or fine tuning said foundational model, the foundational model representing a biological or cellular process; using the foundational model to model a desired biological or cellular process the results of which are used in biological/pharmacological downstream analysis and/or processes.

The computer-implemented method of the first aspect, wherein the real-world gene expression profile dataset is associated with a particular disease, the particular disease comprising a plurality of different disease endotypes, the method further comprising: prior to generating the real-value synthetic gene expression profile dataset, dividing the real-world gene expression profile dataset into a plurality of subsets of the real-world gene expression profile dataset, each real-world gene expression profile sub-dataset associated with a different disease endotype of the particular disease; and generating, for each real-world gene expression profile sub-dataset, a corresponding synthesized gene expression profile sub-dataset in accordance with the computer-implemented method of the first aspect, wherein said outputting data representative of the real-valued synthetic gene expression profile dataset for downstream biological/pharmacological analysis, processing and/or applications further comprising: generating augmented gene expression profile sub-datasets based on storing each real-value synthetic gene expression profile sub-dataset with the corresponding synthetic gene expression profile sub-dataset; generating a training dataset based on labelling each gene expression profile data instance of each augmented gene expression profile sub-dataset according to the corresponding disease endotype; applying the training dataset to a patient / disease stratification machine learning, ML, model for classifying an input gene expression profile of a subject as being associated with one of the disease endotypes of the plurality of different disease endotypes; and performing patient / disease stratification based on applying a plurality of gene expression profiles from a cohort of patients to the trained patient / disease stratification ML model.

The computer-implemented method of the first aspect, wherein said outputting data representative of the real-valued synthetic gene expression profile dataset for downstream biological/pharmacological analysis, processing and/or applications further comprising: generating a training dataset from the real-valued synthetic gene expression profile dataset, wherein the training dataset includes the real-valued synthetic gene expression profile dataset and the corresponding real-world gene expression profiles from the real-world gene expression profile dataset used to generate the real-valued synthetic gene expression profile dataset; iteratively training, using a machine learning algorithm, model parameters of a machine learning, ML, model until a stopping criterion is reached, the ML model configured for predicting / classifying a biological objective and/or pharmacological objective based on the gene expression profiles of the training dataset; performing ML inference by inputting at least data representative of a real-world gene expression profile associated with a subject or data representative of a theoretical gene expression profile to the trained ML model, wherein the trained ML model processes the input real-world gene expression profile or theoretical gene expression profile and outputs a prediction / classification associated with the biological objective and/or the pharmacological objective in relation to the gene expression profiles of the training dataset.

The computer-implemented method of the first aspect, wherein: predicting / classifying the biological objective corresponds to at least one of predicting / classifying cell-type, disease, disease associated with cell-type, and/or any other biological objective; and/or predicting / classifying the pharmacological objective corresponds to at least one of predicting / classifying drug efficacy, drug toxicity, drug and/or drug dosage based on a detected disease, and/or any other pharmacological objective.

The computer-implemented method of the first aspect, wherein the ML algorithm comprises one or more ML algorithms or combinations thereof from the group of: an artificial neural network, NN, based ML algorithm; a decision tree-based classification ML algorithm; a random forest decision tree ML algorithm; an Adaptive Boosting based ML algorithm; a Gradient Boosting based ML algorithm; a Bagging based ML algorithm; an Ensemble based ML algorithm; a large language model; a NN transformer; any other supervised ML algorithm suitable for training model parameters of an ML model for outputting a prediction or classification associated with a biological and/or pharmacological objective associated with the gene expression profiles of the training dataset when data representative of a real-world gene expression profile or a theoretical gene expression profile are input.

The computer-implemented method of the first aspect, wherein said computing the complex gene expression profile dataset further comprising: pre-processing the received gene expression profile dataset to form a normalised gene expression profile dataset; and computing the complex gene expression profile dataset based on applying a discrete Fourier transform to data representative of each normalised gene expression profile of the normalized gene expression profile dataset; and said computing the real-valued synthetic gene expression profile dataset further comprising: applying the inverse discrete Fourier transform to each of the plurality of synthetic complex gene expression profiles to form a normalised synthetic gene expression profile dataset; and post-processing the normalised synthetic gene expression profile dataset based on back translating or reverse-normalising the normalised synthetic gene expression profile dataset into the real-valued synthetic gene expression profile dataset, wherein the real-valued synthetic gene expression profile dataset is a raw representation associated with or in line with the raw representation of the received gene expression profile dataset.

According to a second aspect, there is provided an apparatus comprising a processor, a memory unit and a communication interface, wherein the processor is connected to the memory unit and the communication interface, wherein the processor and memory are configured to implement the computer-implemented method of the first aspect and any one or more features thereof.

According to a third aspect, there is provided a computer program product comprising data or instruction code, which when executed on a processor, causes the processor to implement the computer-implemented method of the first aspect and any one or more features thereof.

According to a fourth aspect, there is provided a computer-readable medium comprising data or instruction code, which when executed on a processor, causes the processor to implement the computer-implemented method of the first aspect and any one or more features thereof.

According to a fifth aspect, there is provided a non-transitory tangible computer-readable medium comprising data or instruction code there is provided a computer-implemented method for generating synthetic gene expression profile datasets for downstream biological/pharmacological analysis, processing and/or applications, which when executed on one or more processors, causes at least one of the processors to perform at least the operations of: receiving a gene expression profile dataset comprising data representative of at least one gene expression profile; computing a complex gene expression profile dataset based on applying a discrete Fourier transform to data representative of each gene expression profile of the received gene expression profile dataset; sampling the complex gene expression profile dataset based on using a statistical distribution to sample and modify a predetermined number of one or more components of each complex gene expression profile for generating a plurality of synthetic complex gene expression profiles; computing a real-valued synthetic gene expression profile dataset based on applying an inverse discrete Fourier transform to each of the plurality of synthetic complex gene expression profiles; and outputting data representative of the real-valued synthetic gene expression profile dataset for downstream biological/pharmacological analysis, processing and/or applications.

According to a sixth aspect, there is provided a computing system comprising one or more processors coupled to a memory, wherein the one or more processors and memory are configured to implement the computer-implemented method of the first aspect and any one or more features thereof.

The computing system of the sixth aspect further comprising: a gene expression profile measurement unit configured for measuring real-world gene expression profiles associated with one or more cells in relation to one or more subjects or cultures; a gene expression profile dataset unit configured for collecting and storing the measured real-world gene expression profiles into one or more gene expression profile datasets according to one or more biological and/or pharmacological criteria; a synthetic gene expression profile generation unit configured for generating a synthetic gene expression profile dataset based on a selected real-world gene expression profile dataset; and a downstream processing unit configured for processing the generated synthetic gene expression profile dataset and/or selected real-world gene expression profile dataset in relation to a biological and/or pharmacological objective or application.

The computing system of the sixth aspect wherein the downstream processing unit further comprising: an ML training module configured for training an ML model for predicting or classifying a biological objective and/or pharmacological objective in relation to the synthetic gene expression profile dataset and/or the corresponding selected real-world gene expression profile dataset; and an ML inferencing module configured for performing inferencing using a trained ML model for predicting or classifying the biological objective and/or pharmacological objective in relation to input data representative of at least a real-world gene expression profile or a synthetic gene expression profile; and an output module configured to output data representative of the predicted or classified biological objective and/or pharmacological objective for further downstream processing in relation to *in vivo* trials, *in vitro* wet lab analysis, and/or high-throughput screening laboratory analysis.

In various implementations, there is provided computer program instructions or code, optionally stored on a non-transitory computer readable medium which, when executed by one or more processors of a data processing apparatus, causes the data processing apparatus to carry out the program instructions to cause the one or more processors to perform operations comprising one or more aspects of the above- and/or below-described implementations (including one or more aspects of the appended claims).

In various implementations, there is provided a computer program product comprising computer program instructions or code which, when executed by one or more processors of a data processing apparatus, causes the data processing apparatus to carry out the program instructions to cause the one or more processors to perform operations comprising one or more aspects of the above- and/or below-described implementations (including one or more aspects of the appended claims).

In various implementations, apparatus are disclosed that comprise a non-transitory computer readable storage medium having program instructions embodied therewith, and one or more processors configured to execute the program instructions to cause the apparatus to perform operations comprising one or more aspects of the above- and/or below-described implementations (including one or more aspects of the appended claims). The apparatus may comprise one or more processors or special-purpose computing hardware.

**Brief Description of the Drawings**

So that the invention may be more easily understood, embodiments thereof will now be described by way of example only, with reference to the accompanying drawings in which:

Figure 1a illustrates an example biological pipeline with synthetic gene expression data generation according to some embodiments of the invention;

Figure 1b illustrates an example synthetic gene expression data generation process according to some embodiments of the invention;

Figure 2 illustrates another example synthetic gene expression data generation process according to some embodiments of the invention;

Figure 3a illustrates an example downstream ML model training process using a training dataset generated from the synthetic gene expression data generation process of Figures 1a to 2 and/or Figures 4a to 8b according to some embodiments of the invention;

Figure 3b illustrates an example patient and/or disease stratification ML process using a training dataset generated from the synthetic gene expression data generation process of Figures 1a to 2 and/or Figures 4a to 8b according to according to some embodiments of the invention;

Figure 3c illustrates an example drug discovery or optimization process using a training dataset generated from the synthetic gene expression data generation process of Figures 1a to 2 and/or Figures 4a to 8b according to some embodiments of the invention;

Figure 4a illustrates a further example synthetic gene expression data generation process according to some embodiments of the invention;

Figure 4b illustrates yet a further example synthetic gene expression data generation process according to some embodiments of the invention;

Figures 5a to 5c illustrate an example methodology for generating synthetic gene expression profiles and performance graphs indicating the synthetic gene expression profiles maintain their cellular identities across different simulated scRNA-seq datasets according to some embodiments of the invention;

Figures 6a to 6e illustrate UMAP visualizations and performance bar plots associated with augmentation of a scRNA-seq PRJEB44878 dataset according to some embodiments of the invention;

Figure 7 illustrates a UMAP representation of the PRJEB44878 dataset with randomly selected individual rare cell types highlighted according to some embodiments of the invention;

Figures 8a to 8b illustrate computational performance comparison as the number of modified CCs and the original dataset sizes increase according to some embodiments of the invention;

Figure 9 is a schematic illustration of a system/apparatus for performing the methods/processes described herein;

Figure 10 is a schematic illustration of a computing system pipeline based on the methods/processes described herein according to some embodiments of the invention.

Common reference numerals are used throughout the figures to indicate similar features.

**Detailed Description**

Various example implementations described herein relate to method(s), apparatus, and system(s) for automatically, efficiently, and reliably generating *in silica* synthetic gene expression profile datasets from real-world gene expression profile datasets for use in downstream biological and/or pharmacological analyses, processes, and/or applications. Gene expression profile data includes, without limitation, for example experimental measurements of the activity or expression of genes of a cellular sample of subject providing a snapshot in time of cellular processes or functions. The cellular sample of a subject may include, without limitation, for example one or more cells, a cluster of cells, an organoid, an organ and/or any cellular portion of the subject. There are several techniques for generating gene expression profile data from cellular samples or cells including, without limitation, for example microarrays for quantifying a set of predetermined sequences or Ribonucleic acid sequencing (RNA-seq) which measures the presence and quantity of RNA molecules, which uses high-throughput sequencing. In any event, analysis and measurement of gene expression of a cellular sample using such techniques, or future sequencing techniques resulting in measurement data representative of the gene expression within the cellular sample, which may be referred to as gene expression profile data. A gene expression profile dataset may include one or more gene expression profile data of a cellular sample of a subject taken at different times and/or one or more gene expression profile data of a cellular sample of one or more subjects. In effect, a gene expression profile dataset includes a plurality of gene expression profiles associated with one or more cellular samples of one or more subjects (e.g., a single cell sample, cell cultures, yeast cultures, bacterial and/or viral cultures and the like). A subject may include, without limitation, for example a person, a patient, a human, a mammal, an animal, a farm animal, a pet, fungi/yeasts/molds and the like, bacteria/bacterium, a virus/virion, a plant, any other living organism, any cell culture, any other suitable subject from which a cellular sample may be produced/extracted for use in gene expression profiling / measuring for generating real-world gene expression profile datasets in relation thereto and the like.

The method(s), apparatus and system(s) for generating synthetic gene expression profile datasets are described herein with reference to, by way of example only but not limited to, gene expression profile datasets associated with single cell RNA-seq (scRNA-seq) performed on one or more cellular samples of one or more subjects and the like. It is to be appreciated by the skilled person that gene expression profile datasets other than scRNA-seq gene expression profile datasets may also be applicable such as, without limitation, for example gene expression profile datasets generated from other RNA-seq and/or microarray techniques and the like, and/or future measurement/sequencing techniques for generating gene expression profile datasets or equivalents thereof, modifications thereto, combinations thereof, and/or as the application demands.

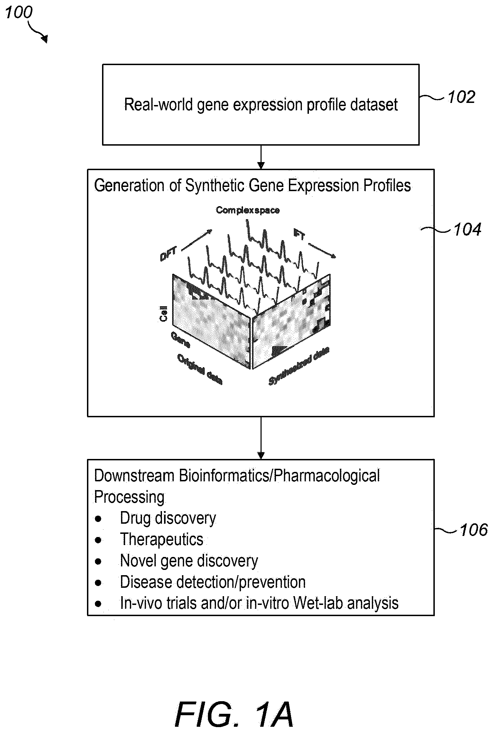

Figure 1a illustrates an example biological/pharmacological pipeline 100 according to some embodiments. The biological/pharmacological pipeline 100 includes a gene profile retrieval component 102, a synthetic gene expression profile generation component 104, and one or more downstream bioinformatics/pharmacological components 106. In this example, the gene profile retrieval component 102 is configured to retrieve a selected real-world or measured gene expression profile dataset for use in downstream processing/analysis. For example, a user of the biological/pharmacological pipeline 100 may select a particular real-world or measured gene expression profile dataset from a gene expression profile database and the like, and/or request measurements/sequencing of cellular samples for generating the relevant real-world gene expression profile dataset. The term real-world is used herein to refer to a gene expression profile dataset that is measured using a desired measurement technique (e.g., scRNA-seq or microarrays) for measuring gene expression of a cellular sample and the like. Once a real-world gene expression profile dataset is selected for subsequent downstream processing/analysis, the real-world gene expression dataset may be augmented or enhanced with *in silico* generated or synthetically generated gene expression profile data using synthetic gene expression profile generation component 104.

The synthetic gene expression profile generation component 104 is configured to receive the selected real-world gene expression profile dataset as input and generate cellular synthetic gene expression profile datasets with similar variability, but which are distinct from the real-world gene expression profile dataset, as an output. The synthetic gene expression profile dataset can be used to augment the real-world gene expression profile dataset for use in downstream analysis/processing.

The synthetic gene expression profile generation component 104 performs a synthetic gene expression profile generation that takes each real-world gene expression profile of the real-world gene expression profile dataset as input and applies the Discrete Fourier Transform (DFT) to map each real-world gene expression profile of the real-world gene expression profile dataset into a vector space referred to herein as a "complex space", in which the transformed real-world gene expression profile dataset is referred to as a real-world complex gene expression profile dataset, which includes one or more real-world complex gene expression profiles. In this complex space, each real-world complex gene expression profile of the complex dataset comprises of a series of complex components (CCs), each of which captures unique modes of variation across genes, which encapsulates the variational elements of the data. The DFT operation is linear and non-reductive, which preserves the full information content of the original input real-world gene expression profile of the input dataset. The synthesis of unique cellular gene expression profiles that are different from the existing real-world gene expression profiles is based on randomly selecting a predetermined number of CCs, which are systematically modified using statistical sampling to generate a plurality of synthesized complex gene expression profiles. The Inverse Discrete Fourier Transform (IFT) is applied to the synthesized complex gene expression profiles to covert the synthesized complex gene expression profiles from the complex space back into the real-world (or real-values) gene expression profile space.

The IFT operation is linear and reconstructive, accurately restoring the full information content of the original data. The IFT globally propagates the impact of the modifications back into the original real-world gene expression profile dataset.

The degree of deviation of each synthesized gene expression profile data from each original real-world gene expression profile data can be adjusted by altering the number of CCs subject to modification. For example, increasing the number of CCs that are modified within the complex space increases the variability of the synthesized gene expression profiles compared with the original real-world gene expression profiles that are input. Consequently, this process enables the controlled synthesis of an endless array of unique cells, each with a nuanced, deviated gene expression profile, while preserving the original cell's gene expression profile variability.

The synthetic gene expression profile generation process of applying a DFT, statistical modification of CCs, and IFT provides distinct advantages over conventional generative ML models for synthesizing gene expression profiles including, for example, being train-free and a cell-centric paradigm. The synthetic gene expression profile generation is computationally efficient and avoids the time-consuming steps associated with training generative ML models that often require graphical processing unit (GPU) and/or specialized AI/ML hardware support and extensive cell counts. Unlike neural network-based generative models, which necessitate careful hyperparameter adjustments, the synthetic gene expression profile generation process as described operates on a stable framework that does not rely on data-specific fine-tuning and optimizations. Finally, the Fourier Transform-based foundation of synthetic gene expression profile generation also does not rely on identifying low-dimensional data manifolds, but instead focuses on capturing the intricacies of individual cell gene expression profiles. The resulting synthetic gene expression profile generation process is a robust alternative for the *in silica* synthesis of, without limitation, for example scRNA-seq gene expression profile datasets and the like. This results in streamlining the data augmentation process and effectively circumventing the common limitations encountered by conventional generative models or manifold techniques.

The resulting synthetic gene expression dataset that is generated by the synthetic gene expression profile generation component 104 may be used to augment the real-world gene expression profile dataset. The augmented real-world gene expression profile dataset, which includes the synthetic gene expression dataset may be used in a range of technical applications including, without limitation, for example patient and/or disease stratification, diagnosis and prognosis, understanding the fundamental biology of disease processes, analyzing and predicting responses to therapeutics and their mechanisms of action, analyzing tissue and cell variability and biology, and endotype, phenotype and biomarker identification. For example, the augmented real-world gene expression profile dataset or the resulting synthetic gene expression dataset may be sent to and/or ingested by a downstream bioinformatics/pharmacological pipeline 106 for use in technical applications performing, without limitation, for example drug discovery; therapeutics; novel gene discovery and applications thereof; disease detection, treatment and/or prevention; analyzing tissue and cell variability and biology, and endotype, phenotype and biomarker identification; training classifiers used in clinical settings including, without limitation, for example patient and disease stratification and endotyping; further downstream analysis including, without limitation, for example mechanisms of actions (MOA) and the like; toxicity and/or efficacy prediction/classification of cellular samples from wet lab analysis or high throughput screening during drug discovery/optimization and testing on said cellular samples; training and fine tuning of biological and/or genomic based foundational models the results of which are applied in subsequent in-vivo trials and/or in-vitro wet lab analysis and other technical biological/pharmacological analyses/processes; and/or for use in subsequent in-vivo trials and/or in-vitro wet lab analysis; and other technical biological/pharmacological analyses/processes and the like.

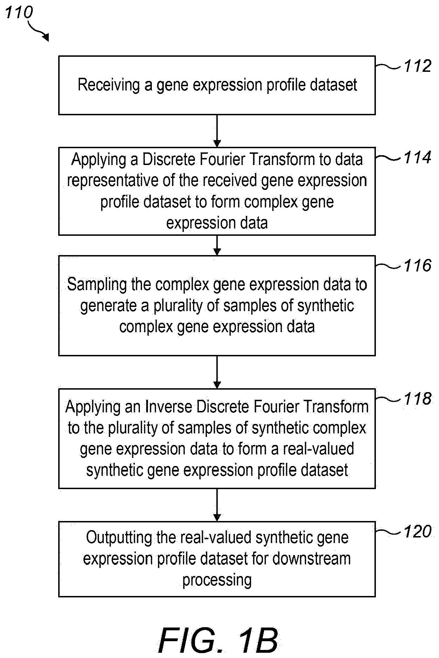

Figure 1b illustrates an example synthetic gene expression data generation process 110 for use in synthetic gene expression profile generation component 104 of Figure 1a. The example synthetic gene expression data generation process 110 includes at least the following steps of:

In step 112, receiving a gene expression profile dataset. The gene expression profile dataset may be a real-world gene expression profile dataset selected by a user for downstream analysis/processing. The received gene expression profile dataset may be derived from real-world measurements of gene expression from cellular samples or one or more cells of at least one subject or source. The gene expression profile dataset may be, without limitation, for example an scRNA-seq generated gene expression profile dataset. In any event, the received gene expression profile dataset may include data representative of at least one gene expression profile.

In step 114, applying a Discrete Fourier Transform (DFT) to data representative of the received gene expression profile dataset to form/compute a complex gene expression profile dataset. This may involve computing a complex gene expression profile dataset based on applying the DFT to data representative of each gene expression profile of the received gene expression profile dataset.

In some embodiments, prior to applying the DFT to the data representative of the received gene expression profile dataset, the received gene expression profile dataset is pre-processed to form a normalised gene expression profile dataset, and thereafter, the complex gene expression profile dataset is computed based on applying the DFT to data representative of each normalised gene expression profile of the normalized gene expression profile dataset. The real-valued synthetic gene expression profile dataset has a raw representation that is associated with or in line with the raw representation of the original received gene expression profile dataset.

For example, prior to applying the DFT to the data representative of the received gene expression profile dataset, each received gene expression profile of the received gene expression profile dataset may be pre-processed from their raw representation and normalised into a common representation. For example, unique molecular identifier (UMI) counts are logarithmically normalized, e.g., for scRNA-seq gene expression profiles, the counts for each cell are scaled to per million counts and then transformed using the natural logarithm.

In step 116, using statistical sampling of the complex gene expression data to generate a plurality of samples of synthetic complex gene expression data. For example, sampling the complex gene expression profile dataset may be based on using a statistical distribution to sample and modify a predetermined number of one or more complex components of each complex gene expression profile for generating a plurality of synthetic complex gene expression profiles. Each complex component of a complex gene expression profile represents a gene associated with the corresponding gene expression profile.

In step 118, applying an Inverse Discrete Fourier Transform (IFT) to each of the plurality of samples of synthetic complex gene expression profiles to compute and form a real-valued synthetic gene expression profile dataset.

In some embodiments, computing the real-valued synthetic gene expression profile dataset may include first applying the IFT to each of the plurality of synthetic complex gene expression profiles to form a normalised synthetic gene expression profile dataset. The normalised synthetic gene expression profile dataset is post-processed based on back translating or reverse-normalising the normalised synthetic gene expression profile dataset to form the real-valued synthetic gene expression profile dataset akin or associated with the original received gene expression profile dataset.

For example, in some embodiments, after applying the IFT to the data representative of the synthetic complex gene expression profile dataset, each real-valued IFT synthetic gene expression profile may be post-processed and back-translated to form a raw real-value synthetic gene expression profile akin to the corresponding original real-world gene expression profile. This post processing may include performing a smoothing process on the real-valued IFT synthetic gene expression profile dataset for mitigating potential noise and/or artifacts introduced during the DFT and/or IFT steps, removing negative values, and taking into account the change rate of the UMI counts between the smoothed and original counterparts.

Alternatively, in other embodiments, after applying the IFT to the data representative of the plurality of samples of synthetic complex gene expression profile dataset, each real-valued synthetic gene expression profile may be post-processed to remove negative values and/or reverse-normalized to a raw gene expression profile representation, taking into account the total UMI count of their original counterparts.

In step 120, outputting the real-valued synthetic gene expression profile dataset for use in downstream analysis/processing. For example, the real-world synthetic gene expression profile dataset may be augmented by the real-valued synthetic gene expression profile dataset to form an augmented gene expression profile dataset for use in downstream analysis/processing.

The downstream biological/pharmacological analysis, processing and/or technical applications may include, without limitation, for example one or more from the group of: *in silica* analysis and processing; drug discovery or optimization; therapeutics; novel gene discovery; disease detection/prevention; *in vivo* trials, *in vitro* wet lab analysis, and/or high-throughput screening laboratory analysis; any other type of technical biological/pharmacological analysis, processing and/or applications using at least the output real-valued synthetic gene expression profile dataset and, optionally, the corresponding real-world gene expression profile dataset; any other type of technical biological/pharmacological processing or applications using at least the output real-valued synthetic gene expression profile dataset and, optionally, the corresponding real-world gene expression profile dataset; and/or any of the biotechnical/technical applications as described herein; modifications thereto; combinations thereof; and/or as the application demands.

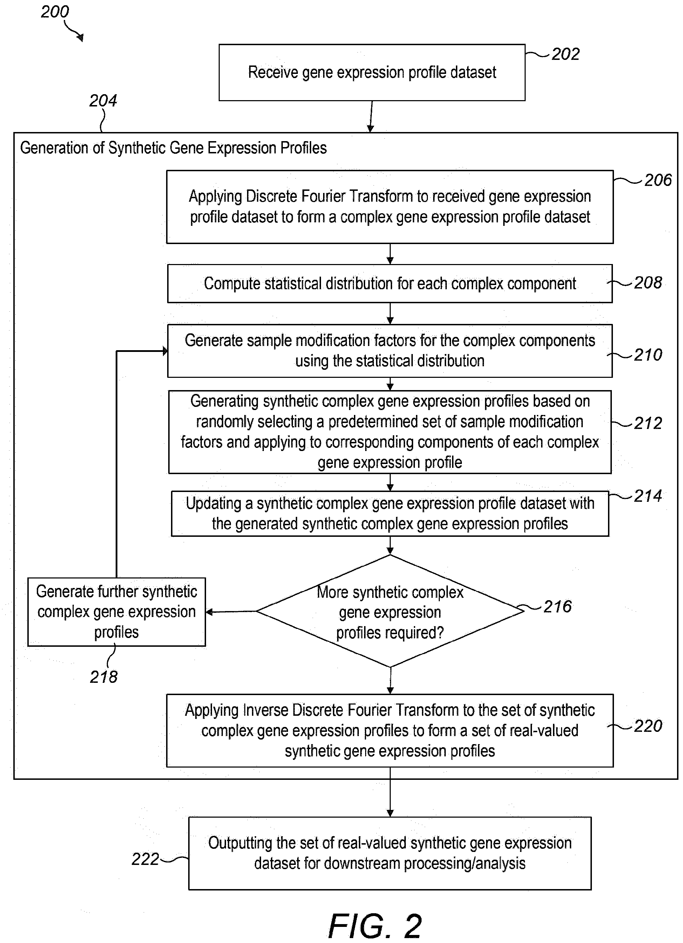

Figure 2 illustrates another example synthetic gene expression data generation process 200 including further modifications to the synthetic gene expression data generation process 110 of Figure 1a. The example synthetic gene expression data generation process 200 includes at least the following steps of:

In step 202, receiving a gene expression profile dataset in a similar manner as described with reference to step 112 of Figure 1b.

In step 204, the received gene expression profile dataset is input to a synthetic gene expression generation component 104 of Figure 1a for processing and generation of a synthetic gene expression profile dataset, in which the generated synthetic gene expression profile dataset is output for downstream processing. Each gene expression profile of the received gene expression profile dataset may be represented as a vector of length $N$, where $N > 0$ is the total number of genes and each element, n, of the vector represents a gene (or gene index) of the gene expression profile for $0 \leq n \leq (N-1)$.

The synthetic gene expression generation component 104 may be further configured to perform the following process steps of:

In step 206, applying a DFT to data representative of each of the received gene expression profiles of the received gene expression profile dataset that is input to form a corresponding complex gene expression profile each of which form a complex gene expression profile dataset.

For example, computing the complex gene expression profile dataset may include applying the DFT to data representative of each gene expression profile of the received gene expression profile dataset. Each complex gene expression profile may be represented as a vector of length $N$, where $N$ is the total number of genes and each element, k, of the vector represents a complex component comprising a complex value with an amplitude and phase, for $0 \leq k \leq (N-1)$.

In some embodiments, prior to applying the DFT to the data representative of the received gene expression profile, each received gene expression profile of the received gene expression profile dataset may be pre-processed from their raw representation and normalised into a common representation. For example, UMI counts are logarithmically normalized, e.g., for scRNA-seq gene expression profiles, the counts for each cell are scaled to per million counts and then transformed using the natural logarithm.

In step 208, computing the statistical distribution for each complex component of the complex gene expression profiles. The statistical distribution is used to generate each sample modification factor for use in modifying each complex component when generating the synthetic complex gene expression profile. For example, in some embodiments this may include computing the mean and variance for each complex component of the complex gene expression profiles of the complex gene expression profile dataset, and selecting a statistical distribution for use in generating sample modification factors for each complex component, in which the selected statistical distribution for each complex component has either the mean and variance computed for that complex component, or a predetermined mean whilst using the variance computed for that complex component.

The statistical distribution may be selected from a probability distribution from the group of: a Gaussian or Normal distribution; a Student's t- distribution; and/or any other suitable symmetric probability distribution for use in evenly distributing enhancement and attenuation modification coefficients.

Computing the statistical distribution may further include determining one or more cell groups for associated with groups of complex gene expression profiles of the complex gene expression profile dataset in which each cell group has a similar or the same gene expression profile. The gene expression profiles of each cell group may meet a similarity threshold or criteria. The statistical distribution is determined for each complex component using the complex gene expression profiles associated with each of the one or more cell groups. For example, the mean and variance of each complex component of the complex gene expression profiles for each cell group of the one or more cell groups may be computed. Each cell group may also be associated with the same cell-type or associated with a cluster of cells and the like.

In some embodiments, when the number of cells in each cell group is greater than 1, then the selected statistical distribution used to generate each sample modification factor of each complex component uses a mean of 2 and the computed variance for said complex component for that cell group. In other embodiments, when the number of cells in each cell group is 1, then the selected statistical distribution used to generate each sample modification factor of each complex component uses a mean of 2 and a variance of 1.

In steps 210 to 218, iteratively performing, until a predetermined number of synthetic complex gene expression profiles have been generated, generation of one or more synthetic complex gene expression profiles. This includes, in step 210, generating a predetermined number of sample modification factors for the complex components using the selected statistical distribution and corresponding mean and variance. There may be different sample modification factors generated for each one or more groups of cells associated with the received gene expression profile dataset. For example, generating the sample modification factors may include generating, by sampling the corresponding statistical distribution for each complex component of each one or more cell groups, sample modification factors corresponding to the N complex components of the complex gene expression profiles for each one or more groups of cells.

In step 212, generating synthetic complex gene expression profiles based on applying, to each complex gene expression profile, a predetermined number of randomly selected sampled modification factors to the corresponding complex components and their conjugate pairs of said each complex gene expression profile. The complex component of each complex gene expression profile that correspond to the Direct Current component is excluded from being randomly selected when performing step 212 and applying sample modification factors.

When there are more than one cell group in the received gene expression profile dataset, this may include generating synthetic complex gene expression profiles for each cell group based on applying, to each complex gene expression profile of each cell group, a predetermined number of randomly selected sampled modification factors to the corresponding complex components and their conjugate pairs of said each complex gene expression profile of said each cell group. For example, a random selection of a predetermined number of complex components is performed and the statistical distribution is used to generate sample modification factors in relation to each of the selected complex components. The random selection of the predetermined number of complex components may be performed using a uniform random probability distribution.

The predetermined number of one or more complex components is selected by a user, in which the variability of the resulting synthetic gene expression profiles in relation to the corresponding real-world gene expression profiles increases as the predetermined number of components increases.

In some example embodiments, when the length of the gene expression profile, $N$, is an even number, then the complex conjugate pair of the $(N/2)$-th complex component is itself and the remaining complex conjugate pair of a $k$-th complex component, for $1 \leq k < N/2$, is the $(N-k)$-th complex component. In other example embodiments, when the length of the gene expression profile, $N$, is an odd number, then the complex conjugate pair of a *k-th* complex component, for $1 \leq k < (N-1)/2$, is the *(N-k)-th* complex component.

In step 214, updating a synthetic complex gene expression profile dataset with the generated synthetic complex gene expression profiles. This may include, when there is more than one cell group, updating the synthetic complex gene expression profile dataset for each cell group with the generated synthetic complex gene expression profiles generated for said each cell group.

In step 216, it is determined whether more synthetic complex gene expression profiles are required. If more synthetic complex gene expression profiles are required, then proceed to step 218, otherwise proceed to step 220. For example, a user may have specified a desired or maximum number of synthetic gene expression profiles are to be generated. Thus, it is determined whether the number of unique synthetic complex gene expression profiles of the synthetic complex gene expression profile dataset will result in the desired or maximum number of synthetic gene expression profiles. If the number of synthetic complex gene expression profiles more synthetic gene expression has not reached the desired maximum number of synthetic gene expression profiles, then proceed to step 218, otherwise proceed to step 220.

In step 218, further synthetic complex gene expression profiles are to be generated, so proceed to step 210 for another iteration of generating synthetic complex gene expression profiles and subsequent update of the synthetic complex gene expression profile dataset.

In step 220, applying an IFT to the plurality of samples of synthetic complex gene expression data for generating data representative of a real-valued normalised synthetic gene expression profile dataset.

In some embodiments, after applying the IFT and generating the real-valued normalised synthetic gene expression profile dataset, each real-valued normalised synthetic gene expression profile may be post-processed and back-translated to form a "raw" real-value synthesized gene expression profile (also referred to as a synthetic gene expression profile) that is akin to the corresponding original real-world gene expression profile. This post processing may include, without limitation, for example performing a smoothing process on the real-valued normalised synthetic gene expression profile dataset for mitigating potential noise and/or artifacts introduced during the DFT and/or IFT steps 206 and/or 220, removing negative values (e.g., ReLU activation function), and taking into account the change rate of the UMI counts between the smoothed and original counterparts.

For example, several post-processing steps may be performed after generating the real-valued normalized synthetic gene expression profile, which includes, without limitation, for example: (1) implementing a smoothing step on the real-valued normalized synthetic gene expression profile dataset for mitigating potential noise and/or artifacts introduced during the DFT/IFT steps 206/220. This may include generating nearest neighbor (NN) graph using the original normalized real-valued gene expression profiles and their nearest neighbors, where the NN graph is applied in smoothing the real-valued normalized synthetic gene expression profiles. For each of the synthesized cells, the corresponding real-valued synthetic gene expression profile values are averaged with those of its original normalized real-valued counterparts and the nearest neighbors determined from the NN graph. This produces a smoothed real-valued normalized synthetic gene expression profile dataset. (2) Since UMIs represent counts of sequences, negative values are biologically implausible. Any negative values that might arise during the DFT/IFT transformation and/or smoothing process are addressed by using a Rectified Linear Unit (ReLU) activation function. The ReLU activation function is applied to each component of each of the smoothed real-valued normalized synthesized gene expression profiles, ensuring all values remain non-negative. This produces a smoothed positive real-valued normalized synthesized gene expression profile dataset. (3) The synthesized raw counts of each smoothed positive real-valued normalized synthesized gene expression profile are derived from their original raw counterparts by taking into account the relative change rate between the smoothed positive real-valued normalized synthesized gene expression profile data and their original normalized counterparts. This outputs the "raw" real-valued synthesized gene expression profile dataset, or final real-valued synthesized gene expression profile dataset, for use in further downstream analysis/processing and the like, and/or as described herein.

Alternatively, in other embodiments, after applying the IFT to the data representative of the complex synthetic gene expression profile dataset, each real-valued normalised synthetic gene expression profile may be post-processed to remove negative values and/or reverse-normalized to a "raw" synthesized gene expression profile representation, taking into account the total UMI count of their original counterparts.

In step 222, outputting the "raw" or final real-valued synthetic gene expression profile dataset for use in downstream analysis/processing. For example, the original real-world synthetic gene expression profile dataset may be augmented by the "raw" or final real-valued synthetic gene expression profile dataset to form an augmented gene expression profile dataset for use in downstream analysis/processing and the like, and/or as described herein.

The "raw" or final real-valued synthetic gene expression profile dataset to form an augmented gene expression profile dataset for use in downstream analysis/processing in many different technical applications including, without limitation, for example disease and/or patient stratification, diagnosis and prognosis, understanding the fundamental biology of disease processes, analyzing and predicting responses to therapeutics and their mechanisms of action, analyzing tissue and cell variability and biology, and endotype, phenotype and biomarker identification, drug discovery and/or optimization and the like. The augmented gene expression profile dataset can be an augmented real-world gene expression dataset that includes both the real-world gene expression profile dataset used to generate the "raw" or final synthetic gene expression dataset and the "raw" or final synthetic gene expression dataset. The augmented real-world gene expression dataset may be used in a range of downstream technical applications in a biological/pharmacological pipeline including, without limitation, for example patient and/or disease stratification, diagnosis and prognosis, understanding the fundamental biology of disease processes, analyzing and predicting responses to therapeutics and their mechanisms of action, analyzing tissue and cell variability and biology, and endotype, phenotype and biomarker identification.

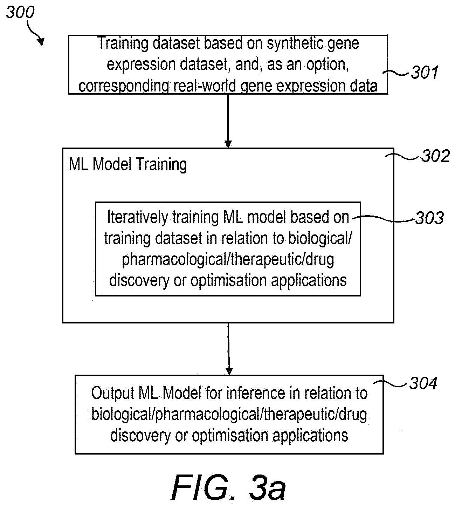

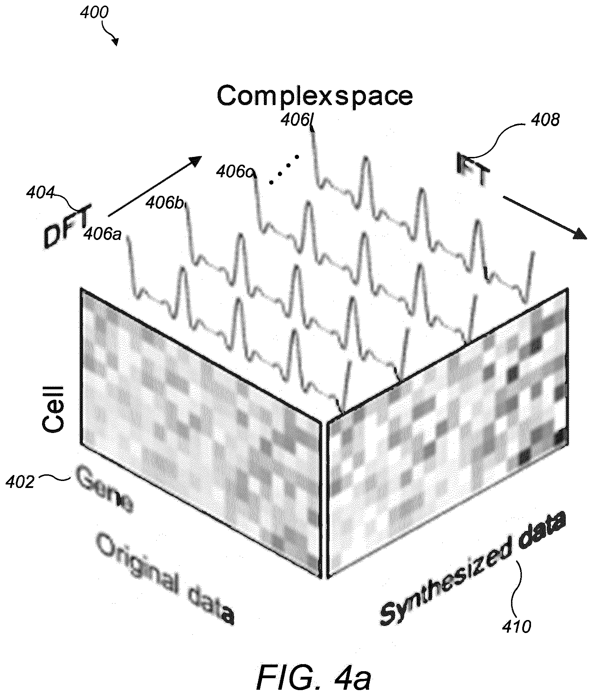

Figure 3a illustrates an example downstream machine learning (ML) model training process 300 that uses a training dataset generated from, for example, the synthetic gene expression data generation processes 110 and 200 of Figures 1a to 2. Once data representative of a "raw" or final synthetic gene expression profile dataset has been output from the synthetic gene expression profile generation component 104 and/or synthetic gene expression profile generation processes 110 and/or 200 with reference to Figures 1a to 2, and/or other processes described herein with reference to Figures 4a to 8b, the synthetic gene expression profile dataset and, as an option, the real-world gene expression profile dataset used to generate the synthetic gene expression profile dataset, is sent to downstream biological/pharmacological components for analysis, processing and/or biotechnical/technical applications as the application demands. That is, an augmented gene expression profile dataset that includes both the real-world gene expression profile dataset and the synthetic gene expression profile dataset may be formed for further downstream processing. In this example, the downstream biological/pharmacological component may include a downstream machine learning component for using the augmented gene expression profile dataset for training one or more ML models / classifiers associated with technical biological and/or pharmacological objectives and technical applications thereto.

It is to be appreciated by the skilled person that an augmented real-world gene expression profile dataset, which includes the synthetic gene expression profile dataset and the real-world gene expression profile dataset used to generate the synthetic gene expression profile dataset using the processes, apparatus, and/or components as described herein, has many technical applications and can be adapted for use in a variety of specific technical bioinformatics/pharmacological applications and implementations thereof. For example, some specific technical bioinformatics/pharmacological applications and implementations include sending, ingesting or applying the augmented real-world gene expression profile dataset in, for example, a downstream bioinformatics/pharmacological pipeline 106 of Figure 1a for use in technical applications such as, without limitation, for example drug discovery; novel gene discovery and applications thereof; disease detection and/or identification; analyzing tissue and cell variability and biology; endotype, phenotype and biomarker identification; training classifiers used in clinical settings including, without limitation, for example patient and disease stratification and endotyping; further downstream processing including, without limitation, for example MOA and the like; toxicity and/or efficacy prediction/classification of cellular samples from wet lab analysis or high throughput screening during drug discovery/optimization and testing on said cellular samples; generating shortlists of candidate molecules / drugs using toxicity and/or efficacy prediction/classification of cellular samples from previous wet lab analysis or high throughput screening during drug discovery/optimization, where the shortlist of candidate molecules/drugs are further tested using in-vivo trials, in-vitro testing; training and fine tuning of biological and/or genomic based foundational models the results of which are applied in subsequent in-vivo trials and/or in-vitro wet lab analysis and other technical biological/pharmacological analyses/processes; *in silica* processing using foundational models that are able to model and/or replace or supplement corresponding downstream processes that use *in vivo* trials, *in vitro* wet lab analysis and assays, high throughput screening, and/or other technical biological/pharmacological analyses/processes; and/or for use in subsequent *in vivo* trials and/or *in vitro* wet lab analysis; and other technical biological/pharmacological analyses/processes and the like.

In this example, the downstream ML model training process 300 may include the following steps of:
In step 301, generating a training dataset from the real-valued synthetic gene expression profile dataset output from the synthetic gene expression data generation processes as described herein, where the training dataset includes the real-valued synthetic gene expression profile dataset and at least the corresponding real-world gene expression profiles from the real-world gene expression profile dataset used to generate the real-valued synthetic gene expression profile dataset. For supervised ML training, the training dataset may be labelled or annotated according to the biological objective and/or pharmacological objective that the trained ML model is to perform when a gene expression profile is input to the trained ML model. For unsupervised ML training, the training dataset is not annotated.

The biological objective may, without limitation, for example correspond to at least one of predicting / classifying cell-type, disease, disease associated with cell-type, patient and/or disease stratification, and/or any other technical biological objective. The pharmacological objective may, without limitation, for example correspond to at least one of predicting / classifying drug efficacy, drug toxicity, drug and/or drug dosage based on a detected disease, and/or any other technical pharmacological objective. The technical biological objective may also be combined with the technical pharmacological objective.

In step 302, supervised or unsupervised ML training is performed based on the generated training dataset. For example, in step 303, iteratively training, using an ML algorithm, model parameters of a ML model until a stopping criterion is reached. The ML model is configured for predicting / classifying a biological objective and/or pharmacological objective based on the gene expression profiles of the training dataset.

In step 304, once the ML model is trained, the ML model may be used in downstream analysis, processing, or applications in relation to further gene expression profiles that may be subsequently measured for input to the trained ML model. For example, the downstream analysis, processing, or applications may include, without limitation, for example performing ML inference by inputting at least data representative of a real-world gene expression profile associated with a subject or data representative of a theoretical gene expression profile to the trained ML model. The trained ML model processes, using the corresponding model parameters, the input real-world gene expression profile or a theoretical gene expression profile and outputs a prediction / classification associated with the biological objective and/or the pharmacological objective of the trained ML model or in relation to the gene expression profiles of the training dataset.

The ML algorithm used for training the model parameters of the trained ML model may include, without limitation, for example one or more ML algorithms or combinations thereof from the group of: a neural network (NN) based ML algorithm; a decision tree-based classification ML algorithm; a random forest decision tree ML algorithm; an Adaptive Boosting based ML algorithm; a Gradient Boosting based ML algorithm; a Bagging based ML algorithm; an Ensemble based ML algorithm; data mining algorithms/methods and/or artificial neural networks (NNs) based algorithms such as, without limitation, for example feed forward NNs, encoder/decoder NNs, recursive NNs, deep NNs, deep learning, deep learning ANNs, convolutional NNs, support vector machines (SVMs), reinforcement learning, large language models, NN transformers, one or more combinations thereof or modifications thereto and the like and/or any other supervised or unsupervised ML algorithm and the like that is suitable for training model parameters of an ML model for outputting a prediction or classification associated with a biological and/or pharmacological objective associated with the gene expression profiles of the training dataset when, for example, data representative of a real-world gene expression profile or a theoretical gene expression profile are input.

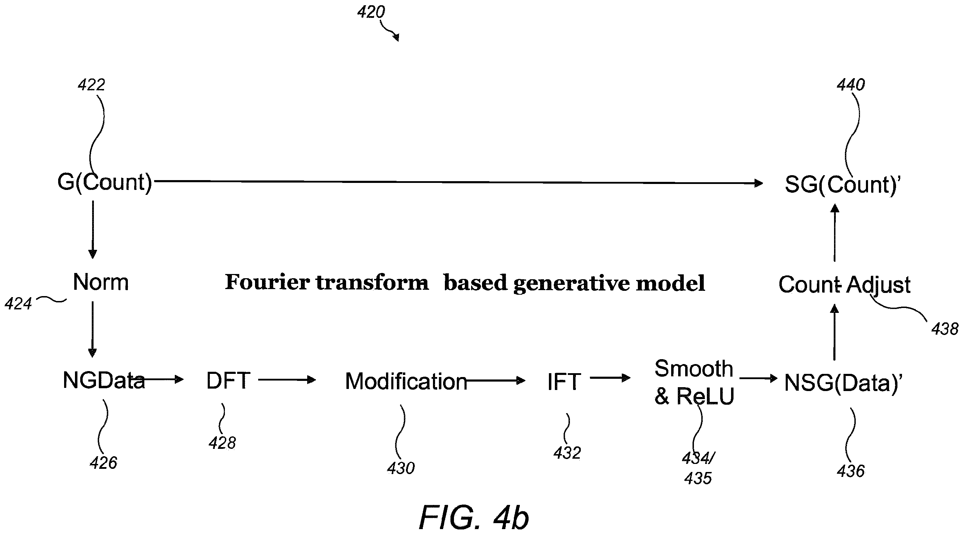

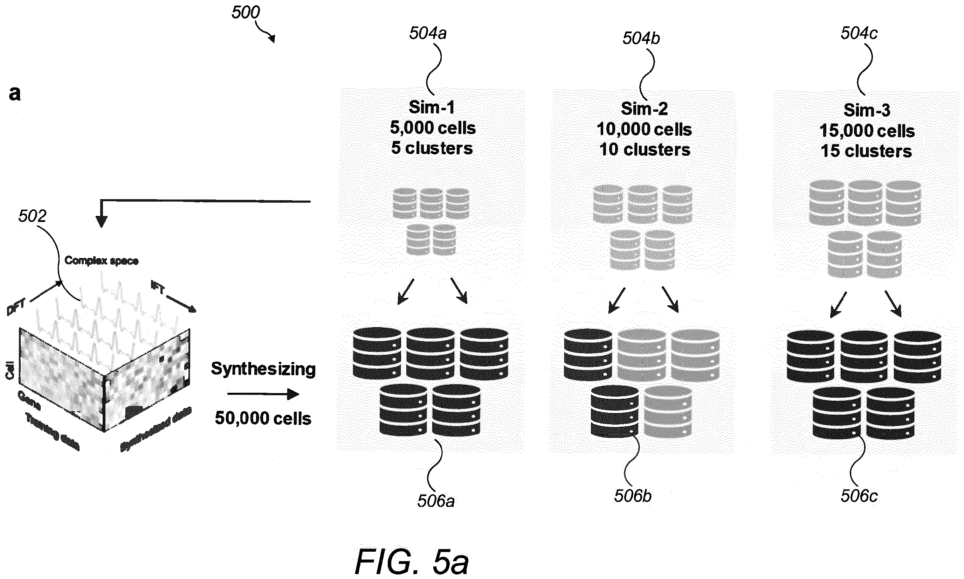

In essence, the synthesized gene expression profile datasets generated from processes 104, 110, 200 of Figures 1a to 2 and/or processes 400 and 420 of Figures 4a and 4b or from scGFT component 502 of Figures 5a to 8b provides the much-needed potential to support the development of newly emerged AI/ML-based technologies, such as foundational ML models for scRNA-seq. An example foundational ML model is described in Cui, H., Wang, C., Maan, H. *et al*. scGPT: toward building a foundation model for single-cell multi-omics using generative AI. *Nat Methods* (2024). https://doi.org/ 10.1038/s41592-024-02201-0, which can be further trained and/or fine-tuned using the synthesized gene expression profile dataset generated by the synthesized gene expression profile processes 104, 110, 200 of Figures 1a to 2 and/or processes 400 and 420 of Figures 4a and 4b and/or scGFT component 502 of Figures 5a to 8b and/or as described herein. An augmented gene expression profile dataset may be used for the training, where the augmented gene expression profile dataset is based on the synthesized gene expression profile dataset and the real-world gene expression profile dataset used to generate the synthesized gene expression profile dataset. The augmented gene expression profile dataset may be applied or input to a foundational model for training or fine tuning said foundational model, in which the foundational model represents or models a biological or cellular process. The trained or fine-tuned foundational model may be used to model a desired biological or cellular process the results of which are used in technical biological/pharmacological downstream analysis, processes and/or applications (e.g., the results are used in *in-silico* processing (e.g., Molecular dynamics processing of compounds etc.), *in vitro* trials/analysis such as wet lab analysis, assays, and/or high throughput screening processes). The augmented gene expression profile dataset is particularly useful in scenarios where training and/or fine-tuning of AI/ML models, classifiers, foundational models for use in biotechnical applications is challenging due to scarce sample gene profile data availability. The augmented gene expression profile dataset can significantly increase the real-world gene profile dataset making it feasible for training and/or fine-tuning AI/ML models, classifiers, foundational models for use in biotechnical or pharmacological processing/applications and the like. By facilitating the generation of anonymized, disease-specific synthetic patient repositories, the synthesized gene expression profile generation processes 104, 110, 200 of Figures 1a to 2 and/or processes 400 and 420 of Figures 4a and 4b, and/or scGFT component 502 of Figures 5a to 8b as described, by way of example, herein are poised to address the financial and ethical constraints associated with clinical settings while supporting the development of robustly trained AI/ML-based models and classifiers essential for the design of clinical trials and/or for drug discovery and optimization, and/or therapeutic discovery and the like.

Some further examples of ML algorithms that may be used to build the AI/ML-based models, classifiers and/or foundational models may include or be based on, by way of example only but is not limited to, one or more ML algorithms associated with random forests, decision tree learning, association rule learning, Adaptive Boosting (AdaBoost) based ML algorithms, Gradient boosting based ML algorithms, extreme Gradient boosting (XGBoost) based ML algorithms, Bagging based ML algorithms, bootstrap aggregating, CoBoost, BrownBoost, data mining algorithms/methods, artificial neural networks (NNs), feed forward NNs, encoder/decoder NNs, recursive NNs, deep NNs, deep learning, deep learning ANNs, convolutional NNs, support vector machines (SVMs), reinforcement learning, large language models, NN transformers, one or more combinations thereof or modifications thereto and the like and/or any other suitable ML algorithm as the application demands. The selection of the hyperparameters for the ML algorithm that affects training the resulting model parameters of the ML model may be based on performing a hyperparameter grid search over a multiple hyperparameter ranges.

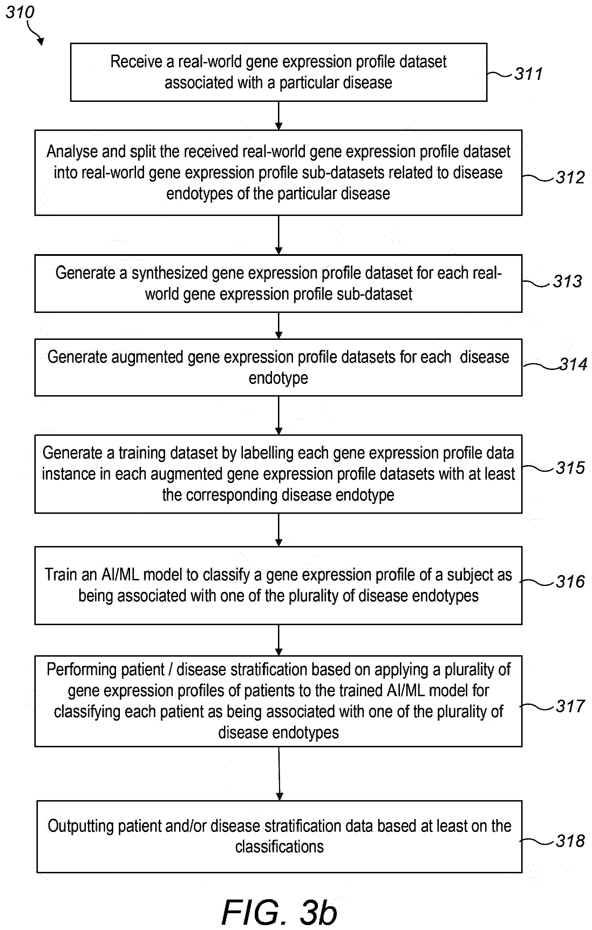

Figure 3b illustrates an example patient and/or disease stratification process 310 that uses training datasets comprising a plurality of augmented real-world gene expression profile datasets, each augmented real-world gene expression profile dataset associated with a particular disease and disease endotype. Disease endotypes may also be referred to as endotypes, which are subtypes of a disease that have different underlying pathobiological mechanisms. The patient and/or disease stratification process 310 is an example process for training classifiers for patient stratification and endotypes. This may be applied at the clinical stage for designing in-vivo trials based on the stratified patients and endotypes. This may be applied at the diagnosis and/or treatment stage for performing patient stratification for a particular disease based on measured gene expression profiles of a cohort of patients.

The example patient and/or disease stratification process 310 may include the following steps of:

In step 311, receiving a real-world gene expression profile dataset associated with a particular disease.

In step 312, prior to generating the real-value synthetic gene expression profile dataset, analyzing the real-world gene expression profile dataset associated with the particular disease to identify those real-world gene expression profiles associated each of a plurality of disease subtype/endotype of the particular disease. For example, the real-world gene expression profile dataset may be split or divided into real-world gene expression profile sub-datasets related to disease endotypes of the particular disease. Each real-world gene expression profile sub-dataset related to a different specific disease endotype of the particular disease. Each real-world gene expression profile sub-dataset for a specific disease endotype of the particular disease including only those real-world gene expression profiles that are identified to be associated with that specific disease endotype.

In step 313, generating a synthesized gene expression profile sub-dataset for each real-world gene expression profile sub-dataset. Each real-world gene expression profile sub-dataset associated with a specific disease endotype is input to, for example, the synthetic gene expression data generation processes 110 and/or 200 with reference to Figures 1a to 2, and/or the other processes 400 and 420 or scGFT component 502 described herein with reference to Figures 4a to 8b, from which a synthetic gene expression profile sub-dataset associated with the specific disease endotype is generated. Each synthetic gene expression profile sub-dataset may have a number of synthetic gene expression profiles suitable for training an ML model (e.g., the number of synthetic gene expression profiles is significantly greater than the number or real-world gene expression profiles in the corresponding real-world gene expression profile dataset). Alternatively, the number of synthetic gene expression profiles of all of the synthetic gene expression profile sub-datasets may be sufficient enough to be suitable for training an ML model.

In step 314, generating an augmented gene expression profile sub-dataset for each specific disease endotype by including from the real-world gene expression profile sub-dataset for said specific disease endotype and the synthetic gene expression profile sub-dataset associated with the specific disease endotype in the corresponding augmented gene expression profile sub-dataset.

In step 315, generating a training dataset by labelling and including each gene expression profile data instance of each augmented gene expression profile sub-dataset with the corresponding disease endotype.

In step 316, using the labelled training dataset with an ML algorithm to train model parameters for a patient /disease stratification ML model to classify an input gene expression profile of a subject/patient as being associated with one of the plurality of specific disease endotype of the particular disease.

In step 317, performing patient / disease stratification based on applying a plurality of gene expression profiles from a cohort of patients to the trained patient / disease stratification AI/ML model for classifying each patient as being associated with one of the plurality of specific disease endotypes. The classification is applied to identify and sort the cohort of patients into groups of patients identified as being associated with a specific disease endotype. Each group of patients including patients identified to be associated with a different specific disease endotype.

In step 318, outputting patient and/or disease stratification data based at least on the classifications and identified groups of patients associated with the specific disease endotypes. Once this is achieved, each group of patients may be further analyzed and/or allocated a treatment protocol that is associated with the specific disease endotype. Alternatively, the gene expression profiles of each group of patients may be input to a further ML model trained to predict a candidate drug or candidate molecule for mitigating the disease endotype.

In another example, the real-world gene expression profile dataset may be associated with gene expression profiles for a particular disease. An augmented gene expression profile dataset for the particular disease may be generated by including the real-world gene expression profile dataset for the particular disease and a synthesized gene expression profile generated by the processes 110, 200, 410, 420 and/or components 502 as described with reference to Figures 1a to 8b using the real-world gene expression profile dataset for the particular disease. Thus, the augmented gene expression profile dataset for the particular disease can be used as a training dataset for use with an ML algorithm for training model parameters of an ML model configured to predict or classify whether an input gene expression profile of a subject / patient is likely to have the particular disease. The trained ML model can be used by inputting a gene expression profile from a cell of the subject / patient, which is processed by the model parameters of the trained ML model and outputs data representative of a prediction or classification of whether the input gene expression profile is associated with the particular disease.

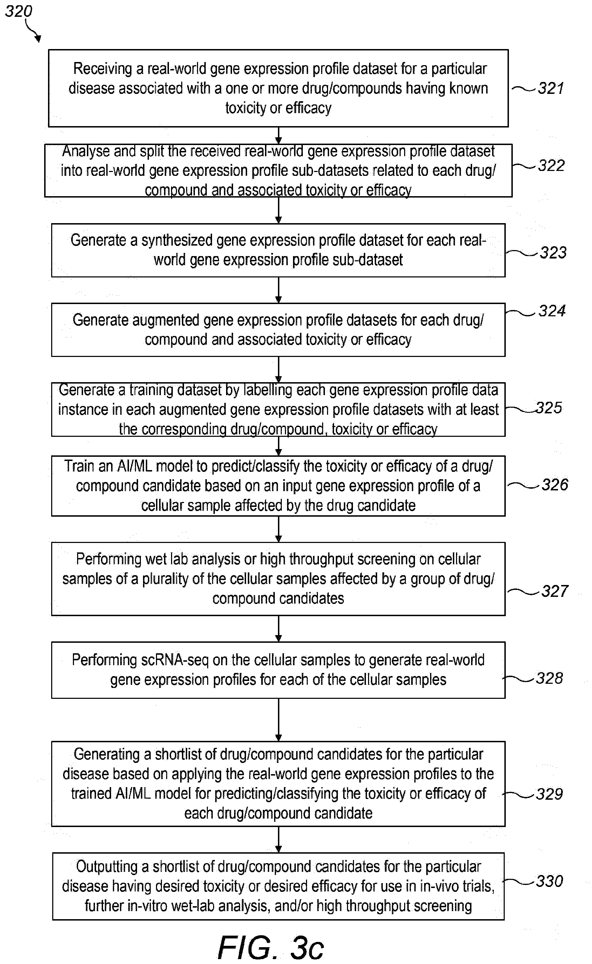

Figure 3c illustrates an example drug discovery or optimization process 320 for a particular disease that uses training datasets comprising a plurality of labelled augmented real-world gene expression profile datasets. The drug discovery or optimization process 320 includes the following steps of:

In step 321, receiving a real-world gene expression profile dataset for a particular disease associated with a one or more drug/compounds having known toxicity or efficacy.

In step 322, analysing and splitting the received real-world gene expression profile dataset for the particular disease into real-world gene expression profile sub-datasets related to each drug/compound and an associated toxicity or efficacy. For example, each real-world gene expression profile sub-dataset is associated with a particular drug/compound having a particular toxicity or efficacy measurement or range of toxicity or efficacy measurements. In an example, each real-world gene expression profile sub-dataset is associated with a particular drug/compound meeting a particular toxicity threshold or efficacy threshold. In another example, each real-world gene expression profile sub-dataset is associated with a particular drug/compound associated with a particular toxicity or efficacy label from a set of labels such as, without limitation, for example a label associated with a low toxicity or low efficacy, a medium toxicity or medium efficacy, or a high toxicity or high efficacy.

In step 323, generating a synthesized gene expression profile dataset for each of the real-world gene expression profile sub-datasets for the particular disease. Each real-world gene expression profile sub-dataset associated with a particular drug/compound having a particular toxicity or efficacy measurement, range, threshold, or label is input to, for example, the synthetic gene expression data generation processes 110 and/or 200 with reference to Figures 1a to 2, and/or the other processes 400 and 420 or components 502 described herein with reference to Figures 4a to 8b, which outputs a synthetic gene expression profile dataset associated with the particular drug/compound having the particular toxicity or efficacy measurement, range, threshold, or label. The user may have specified a desired number of synthetic gene expression profiles that should be generated by the synthetic gene expression processes 110, 200, 400, 420 and/or component 502 etc., i.e., a number that is sufficient for training an AI/ML model. The synthetic gene expression profile dataset may have a number of synthetic gene expression profiles suitable for training an ML model (e.g., the number of synthetic gene expression profiles is significantly greater than the number or real-world gene expression profiles in the corresponding real-world gene expression profile dataset).

In step 324, generating an augmented gene expression profile dataset for each drug/compound having a particular toxicity or efficacy measurement, range, threshold, or label by including the corresponding real-world gene expression profile sub-dataset and the corresponding synthetic gene expression profile dataset for said each drug/compound having the particular toxicity or efficacy measurement, range, threshold, or label.

In step 325, generating a training dataset by labelling each gene expression profile data instance of each augmented gene expression profile dataset with the corresponding drug/compound, toxicity or efficacy measurement, range, threshold, or label, or both toxicity and efficacy measurement, range, threshold, or label. Thus, the training dataset is a training dataset for the particular disease and includes all of the augmented gene expression profile datasets labelled according to the original real-world gene expression profile dataset for the particular disease associated with the one or more drug/compounds having known toxicity or efficacy.

In step 326, using the labelled training dataset with an ML algorithm to train model parameters for a toxicity and/or efficacy AI/ML model to classify / predict the toxicity and/or efficacy of a drug/compound candidate based on an input gene expression profile of a cellular sample affected by the drug/compound candidate.

In step 327, performing wet lab analysis or high throughput screening on cellular samples affected by the particular disease including a plurality of cellular samples affected by the particular disease also affected by a group of drug/compound candidates, with any remaining cellular samples affected by the disease being associated with a control group.

In step 328, performing, without limitation, for example scRNA-seq on the cellular samples to generate real-world gene expression profiles for each of the cellular samples.

In step 329, generating a shortlist of drug/compound candidates for the particular disease based on applying the real-world scRNA-seq gene expression profiles of the drug/compound candidates to the trained AI/ML model for predicting/classifying the toxicity or efficacy of each drug/compound candidate for the particular disease. A drug/compound candidate may be added to the shortlist of drug/compound candidates if the predicted/classified toxicity meets a desired toxicity range/threshold/label or the predicted/classified efficacy meets a desired efficacy range/threshold/label, or both the predicted/classified toxicity and efficacy meet desired ranges/thresholds/labels. For example, the desired toxicity threshold/label may be associated with a maximum acceptable toxicity threshold or label. For example, the desired efficacy range/label may be associated with a minimum acceptable efficacy threshold or label.

In step 330, outputting a shortlist of drug/compound candidates for the particular disease having the desired toxicity and/or desired efficacy for use in in-vivo trials, further in-vitro wet-lab analysis, and/or high throughput screening. The shortlist is used to reduce the number of drug/compound candidates that are to be tested in in-vivo trials, further in-vitro wet-lab analysis, and/or high throughput screening. The above-mentioned process 320 may improve the efficiency of (e.g., speed up) the drug discovery process whilst minimizing laboratory costs in testing a plurality of drug/compound candidates for a particular disease.

Each of the components and/or processes of the biological/pharmacological pipeline 100, and/or synthetic gene expression profile generation processes 110, 200, 410, 420 and/or components 502 as described with reference to Figures 1a to 2 and 4a to 8b and/or downstream processes or applications 300, 310, and/or 320 of Figures 1a to 3c and/or as herein described may be implemented on an apparatus including one or more processors, a memory unit, and a communication interface, in which the one or more processors are coupled to the memory unit and the communication interface. The processor and memory may be configured to implement instructions or code associated with implementing the each of the components of the biological/pharmacological pipeline 100, and/or synthetic gene expression profile generation processes 110, 200, 410, 420 and/or components 502 as described with reference to Figures 1a to 2 and 4a to 8b and/or downstream processes or applications 300, 310, and/or 320 of Figures 1a to 3c and/or as herein described. The processes of the biological/pharmacological pipeline 100, and/or synthetic gene expression profile generation processes 110, 200, 410, 420 and/or components 502 as described with reference to Figures 1a to 2 and 4a to 8b and/or downstream processes or applications 300, 310, and/or 320 of Figures 1a to 3c and/or as herein described may be implemented as computer-implemented methods. The processes of the biological/pharmacological pipeline 100, and/or synthetic gene expression profile generation processes 110, 200, 410, 420 and/or components 502 as described with reference to Figures 1a to 2 and 4a to 8b and/or downstream processes or applications 300, 310, and/or 320 of Figures 1a to 3c and/or as herein described, combinations thereof, modifications thereto and/or further modified based on one or more other processes as herein described with reference to Figures 4a to 8b may comprise one or more computer program product, and/or be stored on a computer-readable medium or non-transitory computer-readable medium. The computer program product may include data or instruction code, which when executed on one or more processors, causes the one or more processors to implement the processes of the biological/pharmacological pipeline 100, and/or synthetic gene expression profile generation processes 110, and 200 and/or downstream processes or applications 300, 310 and/or 320 of Figures 1a to 3c and/or combinations thereof, modifications thereto and/or further modified based on one or more other processes as herein described with reference to processes 410, 420 and/or components 502 as described with reference to Figures 4a to 8b and/or as herein described. The computer-readable medium or non-transitory computer-readable medium may include data or instruction code, which when executed on one or more processors, causes the one or more processors to implement one or more of the processes of the biological/pharmacological pipeline 100, and/or synthetic gene expression profile generation processes 110, 200, 410, 420 and/or components 502 as described with reference to Figures 1a to 2 and 4a to 8b and/or downstream processes or applications 300, 310, and/or 320 of Figures 1a to 3c and/or as herein described and/or combinations thereof, modifications thereto, and/or as the application demands.

Figure 4a illustrates a further example synthetic gene expression data generation process 400 for, without limitation, for example scRNA-seq gene expression profile datasets. synthetic gene expression data generation process 400 performs synthetic gene expression profile generation that takes each real-world gene expression profile of the real-world gene expression profile dataset 402 as input and applies a DFT process 404 to map each real-world gene expression profile of the real-world gene expression profile dataset into a complex vector space, labelled "complex space". The transformed real-world gene expression profile dataset 402 is referred to as a real-world complex gene expression profile dataset, which includes one or more real-world complex gene expression profiles. In this complex space, each real-world complex gene expression profile of the complex dataset includes a series of complex components (CCs), each of which represents a gene of the real-world gene expression profile 402 and captures unique modes of variation across genes, which encapsulates the variational elements of the data. The DFT operation 404 is linear and non-reductive, which preserves the full information content of the original input real-world gene expression profile of the input dataset. The synthesis of unique cellular gene expression profiles that are different from the existing real-world gene expression profiles 402 is based on randomly selecting a predetermined number of CCs, which are systematically modified using statistical sampling to generate a plurality of synthesized complex gene expression profiles 406a-406t. An IFT process 408 is applied to the synthesized complex gene expression profiles 406a-406t to covert the synthesized complex gene expression profiles 406a-406t from the complex space back into the real-world (or real-values) gene expression profile space as synthesized gene expression profile dataset 410. The IFT operation is linear and reconstructive, accurately restoring the full information content of the original data. This means that the IFT globally propagates the impact of the modifications back into the original real-world gene expression profile dataset.

The degree of deviation of each synthesized gene expression profile from each original real-world gene expression profile dataset 402 can be adjusted by altering the number of CCs subject to modification. For example, increasing the number of CCs that are modified within the complex space increases the variability of the synthesized gene expression profiles in the synthesized gene expression profile dataset 410 compared with the original real-world gene expression profiles of the real-world gene expression profile dataset 402. Consequently, this process enables the controlled synthesis of an endless array of unique cells, each with a nuanced, deviated gene expression profile, while preserving the original cell's gene expression profile variability.

Figure 4b illustrates another further example synthetic gene expression data generation process 420. The synthetic gene expression data generation process 420 is based on and further modifies the synthetic gene expression data generation processes 104, 110, 200, 400 as described with reference to Figures 1a to 4a. In this example, the synthetic gene expression data generation process 420 is described with reference to, by way of example only but is not limited to, an scRNA-seq gene expression profile dataset denoted in Figure 4b as G(Count). In this example, the gene expression profile dataset denoted G(Count) or G is generated using scRNA-seq techniques. The gene expression profile dataset G(Count) may include at least one gene expression profile associated with a cell or a plurality of gene expression profiles associated with corresponding cells of one or more subjects. Each of the gene expression profiles G(Count) are quantified for each corresponding cell as a discrete series, $x[n]$, with $n$ indexing genes (0 to $N$ - 1, $N$ being the total genes). The resulting "raw" or final synthetic gene expression profile dataset is denoted in Figure 4b as G(Count)'. The example synthetic gene expression data generation process 420 includes the following steps of:

In step 422, receiving as input a gene expression profile dataset G(Count), where each gene expression profile in the gene expression profile dataset G(Count) is quantified for each cell as a discrete series, $x[n]$, with $n$ indexing genes (0 to $N$ - 1, $N$ being the total genes). Alternatively, each gene expression profile for each cell of the received gene expression profile dataset G(Count) may be represented as a vector of length $N$, where $N > 0$ is the total number of genes and each element, n, of the vector is a gene index that represents a corresponding gene of the gene expression profile for $0 \leq n \leq (N-1)$.

In step 424, pre-synthesis processing (e.g., pre-processing) the input gene expression profile dataset G(Count) in which at least two preliminary processing steps are undertaken to generate a normalized gene expression profile dataset denoted in Figure 4b as NGData prior to performing synthesis using the DFT. For example, in a first preliminary processing step, UMI counts of the gene expression profiles of the input gene expression profile dataset G(Count) are logarithmically normalized, i.e., counts for each cell are scaled to per million counts and then transformed using the natural logarithm. This normalization stabilizes expression variability across cells. In a second preliminary processing step, the synthesis procedure is selectively applied only to the most variably expressed genes in the gene expression profile dataset. These genes, manifesting biological variation are indicative of distinct cellular states or phenotypes, are specifically targeted to mitigate the amplification of stochastic noise prevalent in genes with lower variability.

In step 426, the normalized gene expression profile dataset, denoted NGData, is output from the pre-synthesis processing step 424. For simplicity, each normalized gene expression profile of the normalized gene expression profile dataset NGData is referred to as a discrete series, x[n], with n indexing genes (0 to $N$ - 1, $N$ being the total genes).

In step 428, the output normalized gene expression profile dataset NGData is input to a DFT process in which the DFT is applied to transform each cell gene expression profile of the normalized gene expression profile dataset NGData into the complex space to yield a complex gene expression profile dataset. The complex gene expression profile dataset includes one or more complex gene expression profiles that correspond to the normalized gene expression profiles of the normalized gene expression profile dataset.

For example, the DFT transforms each normalized gene expression profile, $x[n]$, of the normalized gene expression profile dataset NGData into:

$$X[k] = \sum_{n=0}^{N-1} x[n]\, e^{-\frac{i2\pi kn}{N}}, \tag{1}$$

where the sequence $X[k]$ for $0 \leq k \leq (N-1)$ may be referred to as the complex component $k$ of a complex gene expression profile. Here, $X[k]$ denotes the complex value $a + ib$, encapsulating the amplitude, $a$, and phase, $b$, for complex component $k$, with i as the imaginary unit. The IFT can be used to revert the complex space data, $X[k]$, back to the original normalized gene expression profile $x[n]$ based on:

$$x[n] = \frac{1}{N} \sum_{k=0}^{N-1} X[k]\, e^{\frac{i2\pi kn}{N}}. \tag{2}$$

This operation satisfies both additivity and homogeneity principles, and therefore is linear. Additionally, it preserves the order and magnitude of the input gene expression profile.

In step 430, randomly selected complex gene expression profiles of the complex gene expression profile dataset are modified to generate synthetic complex gene expression profiles. The number of randomly selected complex gene expression profiles may be a parameter set by the user and relates to the total number of synthetic gene expression profiles requested or desired. This is achieved by taking advantage of the global impact that complex component modification has on a complex gene expression profile and the resulting real-value gene expression profile after performing an IFT on the complex gene expression profile.

That is, amplitude adjustments to specific complex components (CCs) within a Fourier Transform series of a gene expression profile results in modifications to the gene expression profile during reconstruction via IFT. It has been found that modification of the amplitude of a specific complex component, $X[m]$, within a Fourier-transformed gene expression profile series systematically impacts the entire reconstructed gene expression profile, denoted $x'[n]$.

For example, the modification of the $m$-th complex component, $X[m]$, of a Fourier-transformed series by a multiplicative factor $A$ yields the modified component $AX[m]$. The impact of this alteration on the reconstructed profile, $x'[n]$, as manifested through IFT can be expressed as:

$$x'[n] = \frac{1}{N}\sum_{k=0}^{N-1} X'[k]\, e^{\frac{i2\pi kn}{N}},\qquad (3)$$

where, X'[$k$] denotes the modified CC spectrum. For all $k$ except for $m$, X'[$k$] = $X[k]$; however, for $m$, X'[$m$] = $AX[m]$. The reconstructed signal, $x'[n]$, can thus be expressed as the sum of the original signal (except the m-th component) plus the contribution from the modification:

$$x'[n] = \frac{1}{N}\sum_{k=0\,(k\neq m)}^{N-1} X[k]\, e^{\frac{i2\pi kn}{N}} + \frac{1}{N} AX[m]\, e^{\frac{i2\pi mn}{N}}.\qquad (4)$$

By adding and subtracting the unmodified $m$-th component:

$$x'[n] = \frac{1}{N}\sum_{k=0\,(k\neq m)}^{N-1} X[k]\, e^{\frac{i2\pi kn}{N}} + \frac{1}{N} AX[m]\, e^{\frac{i2\pi mn}{N}} + \left(\frac{1}{N} X[m]\, e^{\frac{i2\pi mn}{N}} - \frac{1}{N} X[m]\, e^{\frac{i2\pi mn}{N}}\right),\qquad (5)$$

the expression can be simplified in the form of:

$$x'[n] = x[n] + \frac{1}{N}(A - 1)X[m]\, e^{\frac{i2\pi mn}{N}}.\qquad (6)$$

From this, it is clear that modifying the complex component $X[m]$ of a complex gene expression profile introduces an additional term to the reconstructed gene expression profile $x'[n]$, which is dependent on $n$, the gene index in the original domain. Thus, the term $(A-1)X[m]e^{i2\pi mn/N}$ encapsulates the impact induced by the alteration of $X[m]$, influencing all values of $n$. This feature demonstrates the systematic propagation of a modification across the entire measured transcriptomic landscape is possible.

Although modifying the amplitude, A, of one or more CCs of a complex gene expression profile changes the resulting gene expression profile after IFT, this needs to be performed in a systematic manner and take into account conjugate symmetry. Conjugate symmetry is a fundamental principle in the application of Fourier Transform techniques to real-valued datasets. Accordingly, any modifications to CCs of a complex gene expression profile must be performed symmetrically. This requirement, along with the inherent conjugate symmetry of the Fourier Transform, ensures the preservation of the data real-valued nature upon performing the IFT on a modified complex gene expression profile. Therefore, when modifying the amplitude of a CC of a complex gene expression profile, it is necessary to apply the same modification to its conjugate pair to maintain the real-valued nature of the resulting gene expression profile after IFT.

For example, given that $x[n]$ is real, the Fourier Transform $X[k]$ of $x[n]$ exhibits a specific symmetry, known as conjugate symmetry, defined by:

$$X[k] = X^*[N - k],\qquad (7)$$

for all $k$, where $X^*[N - k]$ is the complex conjugate of $X[k]$. This is because the DFT of a sequence $x[n]$, where $n = 0l,...,N - 1$ and $N$ represents the total number of genes, is given by:

$$X[k] = \sum_{n=0}^{N-1} x[n]\, e^{-\frac{i2\pi kn}{N}}.\qquad (8)$$

The component $N - k$ can be expressed as:

$$X[N - k] = \sum_{n=0}^{N-1} x[n]\, e^{-\frac{i2\pi n(N-k)}{N}} = \sum_{n=0}^{N-1} x[n]\, e^{-i2\pi n\left(1-\frac{k}{N}\right)} = \sum_{n=0}^{N-1} x[n]\, e^{\frac{i2\pi kn}{N}}.\qquad (9)$$

This equality holds because $e^{-i2\pi n} = 1$ (as $e^{-i2\pi n}$ represents a full rotation in the complex plane). Taking complex conjugate of both sides:

$$X^*[N - k] = \sum_{n=0}^{N-1} x[n] \, e^{-\frac{i2\pi kn}{N}} = X[k].$$

(10)

Here, given the real-valued nature of the gene expression profile, $x^*[n] = x[n]$.

From this, the conjugate symmetry ensures that performing the IFT results in a real-valued signal, adhering to the physical and biological nature of the data represents. For example, given the DFT of a real-valued sequence $x[n]$, denoted by $X[k]$, its IFT is represented as:

$$x[n] = \frac{1}{N} \sum_{k=0}^{N-1} X[k] \, e^{\frac{i2\pi kn}{N}}.$$

(11)

Applying Euler's formula, $e^{i\theta} = \cos(\theta) + i \sin(\theta)$, the contributions of a generic X[k] within the summation is:

$$X[k] \left( \cos\left(\frac{2\pi kn}{N}\right) + i \sin\left(\frac{2\pi kn}{N}\right) \right).$$

(12)

Subsequently, the contribution of its conjugate X[N - k] to the sum, utilizing the conjugate symmetry property, takes the form:

$$X^*[k] \left( \cos\left(2\pi n \frac{N-k}{N}\right) + i \sin\left(2\pi n \frac{N-k}{N}\right) \right).$$

(13)

Given that $X^*[k]$ is the complex conjugate of $X[k]$, if $X[k] = a + ib$, then $X^*[k] = a - ib$. Therefore, both terms include the factor $a \cos(2\pi kn/N)$. Since the cosine function is even ($\cos(-\theta) = \cos(\theta)$), the cosine terms for $k$ and $N - k$ are identical and add together, reinforcing the real component of the IFT. Conversely, the sine terms possess differing signs. The term from $X[k]$ includes:

$$ib \sin\left(\frac{2\pi kn}{N}\right),$$

(14)

while the contribution from X[N - k] comprises:

$$-ib \sin\left(2\pi n \frac{N-k}{N}\right).$$

(15)

Due to sine function odd property ($\sin(-\theta) = -\sin(\theta)$) these sine terms negate each other. Therefore, both terms include only the real factor $-b \sin(2\pi kn/N)$. Hence, adding up the remaining terms from conjugate pairs results in a real value in the form of:

$$2a \cos\left(\frac{2\pi kn}{N}\right) - 2b \sin\left(\frac{2\pi kn}{N}\right).$$

(16)

Thus, it can be seen that maintaining conjugate symmetry when manipulating CCs of a complex gene expression profile ensures that the numerical transformations using IFT preserves the original gene expression profile data real integrity and characteristics.

Maintaining conjugate symmetry may be performed by identifying conjugate pairs for even and odd length gene expression profiles. Applying modifications to CCs of each randomly selected complex gene expression profile involves accurately identifying the gene indices of conjugate pairs, which varies depending on whether the gene expression profile length $N$ (number of detected genes, $N$) is even or odd. For even-length profiles (where $N$ is even), the Nyquist component ($X[N/2]$; indicating the upper limit of complex content) lacks a distinct conjugate pair, i.e., a self-conjugate component. For the remaining components, specifically for $X[k]$ where $1 \leq k < N/2$, the conjugate pair is identified as $X[N - k]$. Both $X[k]$ and corresponding $X[N - k]$ should be modified to uphold conjugate symmetry. For example, in a complex gene expression profile with $N = 6$, $X[1]$ and $X[5]$ form conjugate pairs, as do $X[2]$ and $X[4]$.

In the case of odd-length gene expression profiles (where $N$ is odd), for any component $X[k]$ where $1 \leq k \leq (N-1)/2$, the conjugate pair is $X[N - k]$. Modifications to $X[k]$ should be mirrored in $X[N - k]$ to ensure the preservation of conjugate symmetry. For instance, with $N = 5$, $X[1]$ and $X[4]$ are conjugate pairs, just as $X[2]$ and $X[3]$.

In addition, regardless of the profile length, the 0-th component, i.e., the Direct Current (DC) component, represents the overall average or baseline level of the signal:

$$X[0] = (1/N) \sum_{n=0}^{N-1} x[n].$$ (17)

Given its representation of the baseline transcriptional activity, modifications to this component can disproportionately skew the entire gene expression profile upon transformation back to the original data space. To preserve the intrinsic cellular baseline and ensure that synthetic gene expression profiles maintain a realistic representation of overall gene expression levels, the DC component is kept unaltered. That is, the DC component is not selected for modification using statistical sampling techniques.

The above criteria are applied for modifying the complex components of a complex gene expression profile and generating a synthetic complex gene expression profile and subsequently, after IFT, a synthetic gene expression profile in the gene expression space (e.g., real-valued space). In this example, scRNA-seq gene expression profiles are used. Although scRNA-seq gene expression profiles are described, this is by way of example only, and the skilled person would understand that other types of gene expression profile datasets may also be applicable as the application demands.

Once the synthetic single-cell gene expression profile generation process 410 has commenced by implementing steps 422 to 426 on a single cell gene expression profile dataset, i.e., with the transformation of single-cell gene expression profiles into single cell complex gene expression profiles in the complex space, then step 430 is performed in which modification factors are generated for all CCs and a predetermined number of CCs are stochastically selected for modification. The number of CCs selected for modification affects the variability of the resulting synthetic gene expression profiles compared with the original gene expression profiles from which they are derived.

The modification factors for the CCs are sampled from pre-defined amplitude distributions associated with each CC, but which are aggregated across cell groups having similar gene expression profiles (such as cell-type or cluster of cells). These factors are applied symmetrically to both the selected CCs and their corresponding conjugate pairs to ensure the real-valued nature of the signal is preserved. Subsequently, the IFT is employed to construct synthetic gene expression profiles.

The modification step 430 may be further modified and/or implemented based on the following:

Given gene expression profile dataset G for a population of cells, for each cell, (e.g., in step 428), the DFT is applied to its gene expression profile, $x_j[n]$, to produce a corresponding complex gene expression profile yielding complex components $X_j[k]$, where $n$ indexes genes, $k$ indexes complex component, and j indexes cells:

$$X_j[k] = \sum_{n=0}^{N-1} x_j[n]\, e^{-\frac{i2\pi kn}{N}}.$$ (18)

Next, for each complex gene expression profile for cell j in each cell group and for each complex component $k$, i. e., $X_j[k]$, the mean $\mu_k$ and variance $\sigma_k^2$ of its amplitude are calculated across the cell group:

$$\mu_k = \frac{1}{M} \sum_{j=1}^{M} \widehat{\overline{X}}_j[k],$$

(19)

$$\sigma_k^2 = \frac{1}{M} \sum_{j=1}^{M} \left( \widehat{\overline{X}}_j[k] - \mu_k \right)^2.$$

Here, $M$ denotes the total number of cells in the cell group and $\overline{X}_j[k]$ denotes the amplitude of the $k$-th complex component for the $j$-th cell, $\overline{X}_j[k]$, in the cell group normalized by, without limitation, for example its Euclidean norm (L2 norm) in the form of:

$$\widehat{\overline{X}}_j[k] = \frac{\overline{X_j[k]}}{\left\| \overline{X_j[k]} \right\|_2}$$ (20)

and

$$\overline{X}_j[k] = |a_j[k] + ib_j[k]| = \sqrt{\left(a_j[k]\right)^2 + \left(b_j[k]\right)^2},$$ (21)

respectively.

The modification factor (also referred to herein as a sample modification factor) for each complex component $k$ of a complex gene expression profile in the cell group is calculated based on a statistical distribution. In this example, a Gaussian or Normal distribution is used, for simplicity and by way of example only, but it is to be appreciated that any other suitable symmetric probability distribution may be used. For this example, a Gaussian distribution is then defined for each complex component $k$, of each complex gene expression profile of the cell group and informed by the aggregate statistics from which the modification factor $A_k$ is sampled:

$$A_k \sim \mathcal{N}\left(\mu = 2, \sigma_k^2\right),\ M > 1$$

(22)

$$A_k \sim \mathcal{N}(\mu = 2, 1),\qquad M = 1$$

In this example, the Gaussian distribution is centered at 2, which aims to evenly distribute both enhancement and attenuation modification coefficients. This choice directly corresponds to the mechanism of the propagation modification term, $(A - 1)X[m]e^{i2\pi mn/N}$, as shown in equation (6). If the cell group contains only one cell, the modification factor is sampled from a Normal Gaussian distribution with its mean shifted by +2 units.

Three key aspects are noted regarding the group-based nature of the synthesis process: (1) Each CC encapsulates a unique variation pattern that, despite varying in magnitude and phase across cells, captures modes of variation present in groups of cells with similar gene expression profiles, such as specific cell types or clusters. As well, equation (22) has been formulated to mathematically embody this concept. (2) To ensure proportional expansion across different cell populations, all pre-defined cell groups are uniformly scaled according to the desired expansion magnitude. This approach maintains the relative proportions among cell groups, ensuring that the expanded populations accurately reflect the original abundance distribution. (3) For a fully stochastic synthesis process, cells are randomly selected for synthesis within each group.

The sampled modification factors are then applied to a predetermined number of randomly selected k-th complex components and their conjugate pairs. For example, for a selected k-th complex component of a randomly selected complex gene expression profile with complex components, X[k], the modified k-th complex component and corresponding conjugate pair is generated based on applying the k-th sampled modification factor, $A_k$, as follows:

$$X'[k] = A_k X[k],$$

(23)

$$X'[N - k] = A_k X[N - k].$$

This is performed for each of the predetermined number of randomly selected complex components. This may be performed on each of the M complex gene expression profiles for each cell group, to generate M synthetic complex gene expression profiles. Should additional synthetic complex gene expression profiles be required, then modification step 430 may be iterated with a different sampling using the statistical distribution and different sampling of the predetermined randomly selected CCs until the required number of synthetic complex gene expression profiles have been generated to form a synthetic complex gene expression profile dataset. Once this is achieved, the process 420 may proceed to step 432.

In step 432, each of the synthetic complex gene expression profiles with adjusted complex components, $X'$, of the synthetic complex gene expression profile dataset are transformed back into the gene expression space to construct the corresponding synthetic gene expression profile for a new cell based on applying the IFT to said each synthetic complex gene expression profile as follows:

$$x'[n] = \frac{1}{N} \sum_{k=0}^{N-1} X'[k]\, e^{\frac{i2\pi kn}{N}},$$

(24)

where x'[n] represents the gene at the n-th gene index of the real-valued normalized synthetic gene expression profile for $0 \leq n \leq N$ and $N$ being the total number of genes and X'[k] is the k-th complex component of the corresponding synthetic complex gene expression profile for $0 \leq k \leq (N-1)$. The IFT is performed on each of the synthetic complex gene expression profiles of the synthetic complex gene expression profile dataset and outputs a real-valued normalized synthetic gene expression profile dataset.

After applying the IFT to the synthetic complex gene expression profile dataset and generating the real-valued normalised synthetic gene expression profile dataset, each real-valued normalised synthetic gene expression profile may be post-processed and back-translated to form a "raw" or final synthetic gene expression profile dataset G(Count)' 440 that is akin to the corresponding original real-world gene expression profile data G(Count) in step 422 used during the synthesis process 420. This post processing may include, without limitation, for example performing a smoothing process on the real-valued normalised synthetic gene expression profile dataset for mitigating potential noise and/or artifacts introduced during the DFT and/or IFT steps 428 and/or 432, removing negative values (e.g., ReLU activation function or other function configured for removing negative values), and taking into account the change rate of the UMI counts between the smoothed and original counterparts from which the "raw" or final synthetic gene expression profile G(Count)' 440.

Several post-synthesis processing steps 434 to 438 are undertaken after IFT step 432 to generate the "raw" or final synthetic gene expression profile G(Count)' 440. In step 434, which is the first processing step (1), smoothing of the real-valued normalized synthesized gene expression profiles is performed for use in mitigating potential noise and/or artifacts that may be introduced during the IFT and/or DFT processes of steps 432 and/or 428. The step 434 leverages the original normalized real-valued gene expression profiles NGData 426 and their nearest neighbors, as defined by a nearest neighbor (NN) graph. For each synthesized cell, the real-valued normalized synthetic gene expression profile values are averaged with those of its original real-valued normalized gene expression profile counterpart and their nearest neighbors. The first processing step 434 outputs smoothed normalized synthesized gene expression profiles. In step 435, which is the second processing step (2), since UMIs represent counts of sequences, negative values are biologically implausible, which is addressed in step 434 by using, without limitation, for example a Rectified Linear Unit (ReLU) activation function. The ReLU activation function is applied to each component of each of the smoothed normalized synthesized gene expression profiles output from step 434 (i.e., the first processing step (1)). This ensures all values of each of the smoothed normalized synthesized gene expression profiles remain non-negative. For example, the ReLU activation function used may be based on:

$$x'_{ReLU}[n] = \max(0, x'[n]), \tag{25}$$

where $x'_{ReLU}[n]$ is the n-th positive real-valued normalized synthetic gene expression profile. Thus, any negative values that might arise during the IFT transformation process of step 432 are removed from the smoothed normalized synthesized gene expression profiles to output a smoothed positive real-valued normalized synthetic gene expression profile dataset NSG(Data)' 436. In step 438 (i.e., the third processing (3)), the synthesized raw counts of each smoothed positive real-valued normalized synthetic gene expression profile of the dataset NSG(Data)' 436 are derived from their original raw counterparts by taking into account the relative change rate between each smoothed positive real-valued normalized synthesized gene expression profile and their original normalized counterparts. The resulting output is a "raw" or final synthesized gene expression profile dataset G(Count)' 440 for use in further downstream analysis/processing and the like, and/or as described herein.

In an alternative embodiment, after applying the IFT to the synthetic complex gene expression profile dataset and generating the real-valued normalised synthetic gene expression profile dataset, each real-valued normalised synthetic gene expression profile may be post-processed and back-translated to form a "raw" or final synthetic gene expression profile dataset G(Count)' in step 440 that is akin to the corresponding original real-world gene expression profile data G(Count) in step 422 used during the synthesis process 420. This post processing may include, without limitation, for example a post-synthesis processing that includes two processing steps that replace the above-mentioned three processing steps 434-438. In the first processing step of the two processing steps, since UMIs represent counts of sequences, negative values are biologically implausible. This is addressed using a ReLU activation function (e.g., equation (25)) which is applied to each component of each of the synthesized gene expression profiles output from the IFT step 432, which ensures all values of each of the synthesized gene expression profiles remain non-negative. Thus, any negative values, which might arise during the IFT and/or DFT transformation processes of steps 432 and/or 424, are removed from the synthesized gene expression profiles to form a normalized positive synthetic gene expression profile dataset. In the second processing of the post-synthesis processing with two processing steps, the normalized positive synthetic gene expression profile dataset is reverse-normalized to form a "raw" of final synthesized gene expression profile dataset or representation by taking into account the total UMI count of their original counterparts. This outputs the "raw" or final synthesized gene expression profile dataset G(Count)' in step 440 for use in further downstream analysis/processing and the like, and/or as herein described.

Although the above-mentioned pre-processing methods/techniques for normalizing the original real-world gene expression profile datasets are described herein, this is by way of example only and the pre-processing method/technique is not so limited, it is to be appreciated by the skilled person that any other pre-processing method/technique for normalizing the original real-world gene expression profile datasets to form a normalized real-world gene expression profile dataset for further processing (e.g., DFT, sampling/synthesis and IFT) may be applied/applicable as the application demands. Although the above-mentioned post-processing methods/techniques for back-translating or reverse-normalizing the normalized synthesized gene expression profile dataset output from the IFT are described herein, this is by way of example only and the post-processing methods/techniques are not so limited, it is to be appreciated by the skilled person that any other post-processing method for back-translating or reverse normalizing the normalized synthesized real-valued gene expression profile datasets into a "raw" synthesized gene expression profile dataset akin to the corresponding original real-world gene expression profile dataset may be applied as the application demands.

Overall, the above process 420 describes the generation of synthetic scRNA-seq gene expression profile datasets by introducing controlled variations, thereby simulating the natural biological variability inherent in authentic datasets. The degree of deviation of the synthetic gene expression profiles from the original gene expression profiles is modulated / controlled by varying the number of CCs that are modified. That is, a user or automated program, depending on the application, may specify a predetermined number of CCs that are to be modified to achieve a particular degree of deviation. This provides a systematic method for controlling the variability introduced into the synthetic gene expression profiles. As the number of CCs increase so does the degree of deviation of the synthetic gene expression profiles from the original gene expression profiles.

Figures 5a to 8 illustrate examples of the performance of the synthetic gene expression data generation process(es) / component(s) 104, 110, 200, 400, and/or 410 of Figures 1a to 4b for generating synthesized gene expression profiles from real-world gene expression profiles from original cells. The fidelity of the resulting synthetic gene expression profiles in mimicking the transcriptional characteristics of the original cells can be evaluated/assessed in which the deviations between the real-world gene expression profiles of the original cells and their synthesized counterparts can be quantified based on the following:

Let $O = (o_1, o_2, ... , o_N)$ represent the gene expression profile of an original cell, where $N$ is the total number of genes and $o_g$ indicates the expression level of gene $g$ in the original cell. Similarly, let $S^i = (s_1^i, s_2^i, ... , s_N^i)$ denote the synthetic gene expression profile of the i-th synthesized cell derived from $O$, with $i \in \{1,2, ... , L\}$ representing the index of synthesized cells, where $L$ is the total number of synthesized cells derived from 0. Here, $s_g^i$ indicates the synthesized gene expression level of gene $g$ in the $i$-th synthesized cell. Then, the difference in gene expression for gene $g$ between the original cell and each synthesized cell is computed as:

$$\Delta_g^i = s_g^i - o_g \qquad (26)$$

The average deviation per gene across all synthesized cells is then calculated as:

$$\overline{\Delta}_g = \frac{1}{L} \sum_{i=1}^{L} \Delta_g^i. \qquad (27)$$

This measure provides an indication of how closely the synthetic gene expression profiles of the synthetic cell resemble that of the original cell, which can be used for assessing the fidelity and/or impact of the synthesize process on the original cell transcriptional landscape.

As mentioned above, synthetic gene expression data generation process(es) / component(s) 104, 110, 200, 400, and/or 410 of Figures 1a to 4b for generating synthesized gene expression profiles from real-world single cell (sc) RNA-seq gene expression profile data from original cells. The publicly available real-world scRNA-seq gene expression profile dataset from Project: PRJEB44878, a study of scRNA-seq based characterization of smoke effects on small airway epithelial cells (SAEC) is used to verify the fidelity of the synthesized gene expression profiles generated based on the synthetic gene expression data generation process(es) / component(s) 104, 110, 200, 400, and/or 410 of Figures 1a to 4b. It was found that the synthetic gene expression data generation process(es) preserves distinct cellular identities in simulated data during the synthesis process resulting in a synthesized gene expression profile dataset that accurately mimics the transcriptional characteristics of the original cells, i.e., the original real-world gene expression profile dataset (in this case, the PRJEB44878 scRNA-seq dataset).

The PRJEB44878 scRNA-seq dataset is processed based on using the CellBridge workflow starting from FASTQ files as outlined in Nouri, Nima, Andre H Kurlovs, Giorgio Gaglia, Emanuele de Rinaldis, and Virginia Savova, 2023, "Scaling up single-cell RNA-seq data analysis with CellBridge workflow.", Bioinformatics (Oxford University Press) 39 (12): btad760. The FASTQ files are available in the European Nucleotide Archive database under accession code PRJEB44878. Canonical QC parameters were employed throughout the processing steps, including the minimum number of UMIs per cell (set to 750), the minimum number of genes per cell (set to 250), the minimum number of cells expressing a given gene (set to 3), the percentage of mitochondrial genes per cell (set to 15), and the threshold used for doublet identification (set to 0.25). Default parameters were used unless otherwise specified.

Pre-processing of the PRJEB44878 scRNA-seq dataset, real-world scRNA-seq gene expression profile dataset, included adhering to the standard Seurat pipeline (v5.0.1) for scRNA-seq data processing, which is outlined in Hao, Yuhan and Stuart, Tim and Kowalski, Madeline H and Choudhary, Saket and Hoffman, Paul and Hartman, Austin and Srivastava, Avi and Molla, Gesmira and Madad, Shaista and Fernandez-Granda, Carlos and others,"2024, "Dictionary learning for integrative, multimodal and scalable single-cell analysis.", Nature biotechnology (Nature Publishing Group US New York), 42 (2), pages 293--304. In addition, data normalization was first carried out using the "NormalizeData" function with the "LogNormalize" method and a scale factor of $10^6$. This was followed by identifying the top 2,000 variable features using "FindVariableFeatures". The data were then centered and scaled using "ScaleData" to ensure uniformity across features. Principal Component Analysis (PCA) was executed with "RunPCA". To adjust for batch effects in the experimental data, the Harmony (v1.2.0) algorithm as outlined in Korsunsky, Ilya and Millard, Nghia and Fan, Jean and Slowikowski, Kamil and Zhang, Fan and Wei, Kevin and Baglaenko, Yuriy and Brenner, Michael and Loh, Po-ru and Raychaudhuri, Soumya, 2019, "Fast, sensitive and accurate integration of single-cell data with Harmony.", Nature methods (Nature Publishing Group US New York) 16 (12): pages 1289-1296 was applied via "RunHarmony" on PCA-reduced data, considering sample-specific variations. Neighbors were identified using "FindNeighbors" based on the harmonized components, which facilitated the clustering of cells into distinct groups with "FindClusters" using a resolution of 0.7.

The fidelity and assessment of the combined datasets of original and synthetic gene expression profile datasets (e.g., original and synthetic cells) underwent another round of features identification, scaling, PCA, batch correction (considering both sample- and synthesis-specific variations), neighbor identification, and clustering. Finally, the integrated data landscape was visualized using "RunUMAP". Unless otherwise specified, default parameters were used for the various standard analysis/processing functions (e.g., "NormalizeData" function, "LogNormalize" method, "RunHarmony", "FindNeighbors", "FindClusters", "RunPCA", "RunUMAP", and the like) as described herein.

In the simulated data processing, cells with fewer than 250 detected genes and genes expressed in fewer than 500 cells were excluded. Normalization (count per million) was performed, and highly variable genes were identified using the standard Seurat pipeline. The combined dataset of original and synthetic cells underwent another round of features identification, scaling, PCA, and batch correction, accounting for synthesis-specific variations. For clustering the integrated data, the k-means approach was adopted to align the number of clusters with the original dataset, ensuring a conservative comparison by equating the grounds. This process was executed using the "kmeans" function from the "stats" package (v3.6.2), applied to the batch-corrected embeddings. Default parameters were used, unless otherwise specified.

Figures 5a-5c illustrates the methodology for generating synthetic gene expression profiles and graphs showing that the cells or synthetic gene expression profiles synthesized by the scGFT component/function maintains their cellular identities across different simulated scRNA-seq datasets. The quality of cells synthesized by the scGFT component, which is implemented based on based on the synthetic gene expression data generation process(es) / component(s) 104, 110, 200, 400, and/or 410 as described with reference to Figures 1a to 4b and/or as herein described, is evaluated with a focus on the fidelity of synthesized gene expression profiles to their original counterparts. To achieve this, clustering analysis was employed to rigorously examine entire gene expression profiles, followed by calculating the likelihood that synthesized cells would cluster with their original counterparts. ScRNA-seq simulated datasets were used to effectively benchmark this criterion, laying a solid groundwork for the analysis.

Figure 5a is a schematic diagram illustrating an experimental system and methodology 500 that was employed to evaluate the quality and fidelity of the scGFT component 502, which is implemented based on based on the synthetic gene expression data generation process(es) / component(s) 104, 110, 200, 400, and/or 410 as described with reference to Figures 1a to 4b and/or as herein described, to ingest three groups of real-world original gene expression profile datasets 504a-504c (e.g., Sim-1, Sim-2, Sim-3) corresponding to a different number of original cells (e.g., 5,000 cells, 10,000 cells, 15,000 cells) and generate three groups of synesthesia gene expression profile datasets 506a-506c, each of which include 50000 synthesized cells or synthesized gene expression profiles.

Three distinct sets of scRNA-seq data were used in the evaluation, each varying in size and complexity. These included: (1) five datasets 504a, each containing 5,000 cells distributed into five clusters; (2) five datasets 504b, each with 10,000 cells spread across ten clusters; and (3) five datasets 504c, each comprising 15,000 cells divided into fifteen clusters. Cells within each cluster were randomly assigned following a uniform distribution, with each dataset consisting of 10,000 genes. For each of these simulated datasets, 50,000 cells were synthesized (i.e., 50000 synthesized gene expression profiles) to evaluate the scalability and robustness of the scGFT component 502 and methodology.

In particular, the three groups of synthesized gene expression profile datasets 506a-506b are generated using scGFT component 502 each with a different input configuration, which include: (1) the synthesized gene expression profile dataset 506a is generated using the scGFT component 502 from five real-world gene expression profile datasets 504a, each containing 5,000 cells divided into five clusters (sim-1); (2) the synthesized gene expression profile dataset 506b is generated using the scGFT component 502 from five real-world gene expression profile datasets 504b, each with 10,000 cells across ten clusters (sim-2); and (3) the synthesized gene expression profile dataset 506c is generated using the scGFT component 502 from five real-world gene expression profile datasets 504c, each comprising 15,000 cells sorted into fifteen clusters (sim-3). Each cluster within these datasets was populated through random assignment following a uniform distribution, with each dataset featuring 10,000 genes. In addition, multiple experiments were performed to generate multiple datasets for each of the input datasets 504a-504c, from which 50,000 cells were synthesized or synthesized gene expression profiles generated, based on varying the number of complex components that were modified. In each experiment for each input dataset 504a-504c a different number of complex components (CCs) was selected based on the values 5, 10, 25, 50, 75, and 100.

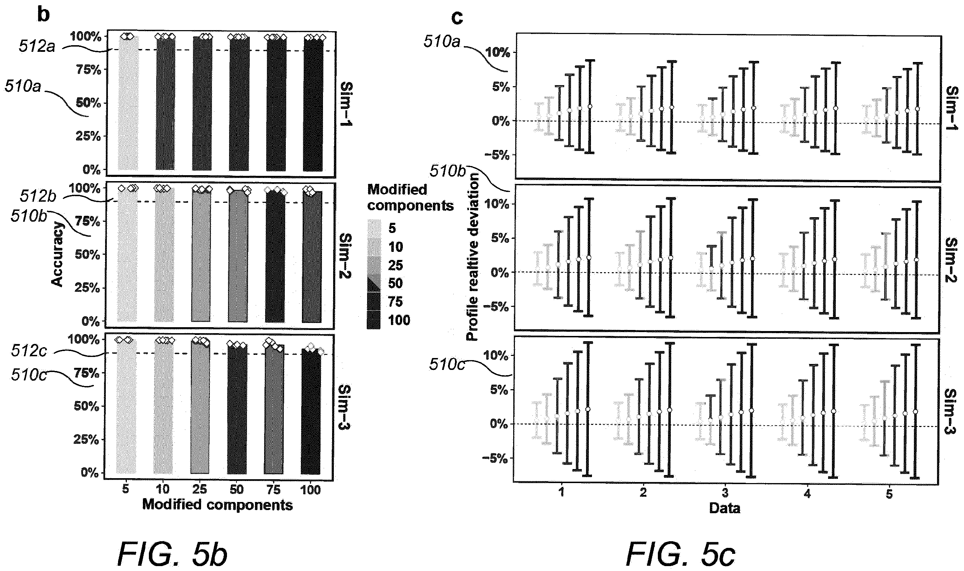

Synthesis was performed (n=50,000 cells) for each dataset 506a-506c, with varying numbers of CCs (5, 10, 25, 50, 75, and 100) modified to examine the impact that different levels of modification on synthesis fidelity. Synthesized cells were then integrated with the original data and re-clustered. The quality of the synthesis process was assessed by calculating the proportion of synthesized cells that correctly clustered with their corresponding original cells. This evaluation revealed a high accuracy, exceeding 92% in each dataset and averaging above 97% across all numbers of modified CCs and simulated data as illustrated in Figure 5b.

Figure 5b illustrates a set of bar plots 510a-510c depicting the proportion of synthesized cells that accurately clustered with their corresponding original cells, for each dataset (diamonds). The x-axis represents the number of complex components modified, and the y-axis shows the clustering accuracy. Bar plot 510a corresponds to the multiple synthesized gene expression profile datasets 506a, bar plot 510b corresponds to the multiple synthesized gene expression profile datasets 506b, and bar plot 510c corresponds to the multiple synthesized gene expression profile datasets 506c. Each row corresponds to one of the three groups of datasets. A horizontal dashed line 512a-512c highlights the 90% accuracy threshold.

As can be seen in bar plot 510c, there is a decrease in accuracy in the analysis of the third simulated dataset 506c (i.e., Sim-3 with 15 clusters) as the number of modified CCs increased. The impact of these modifications appears to correlate with the complexity of the dataset, where this trend is absent in datasets 506a and 506b of bar plots 510a and 510b with fewer cells and clusters (5 and 10 clusters). As can be seen, the number of CCs, which is a user input to the scGFT component 502, affects the extent of deviation introduced through CC modification, which if too large, can excessively distance the synthesized cells' gene expression profile from their original counterparts.

Figure 5c illustrates error bar plots 520a-502c with error bars (mean $\pm$ standard deviation) depicting the impact of modifying varying numbers of complex components on the deviation of gene expression profiles in synthesized cells from their original counterparts (y-axis), for different datasets (x-axis). Each row corresponds to one of the three groups of datasets 502a-502c. Error bar plot 520a corresponds to the synthesized gene expression profile datasets 506a, error bar plot 520b corresponds to the multiple synthesized gene expression profile datasets 506b, and error bar plot 520c corresponds to the multiple synthesized gene expression profile datasets 506c.

The error bar plots 520a-520c illustrate the impact of altering the number of modified CCs on the divergence of the synthesized gene expression profiles between synthesized cells and their original cells was assessed by calculating the relative average deviation of gene expression between synthesized and original cells. These results demonstrate an anticipated increase in deviation magnitude as more CCs are modified, aligning with the theoretical underpinnings of the scGFT methodology / component 502. Moreover, there is a slight positive shift in the deviation distributions. This shift is attributed to implementation of the Rectified Linear Unit (ReLU) function, which clips negative values introduced during synthesis to zero in the post processing processes of the scGFT component.

The experimental system and methodology 500 of Figures 5a-5c confirms the controlled basis of the variations introduced by scGFT component 502 and also the biological plausibility of synthesized cells by maintaining the distinct cellular identities in proximity to their original counterparts.

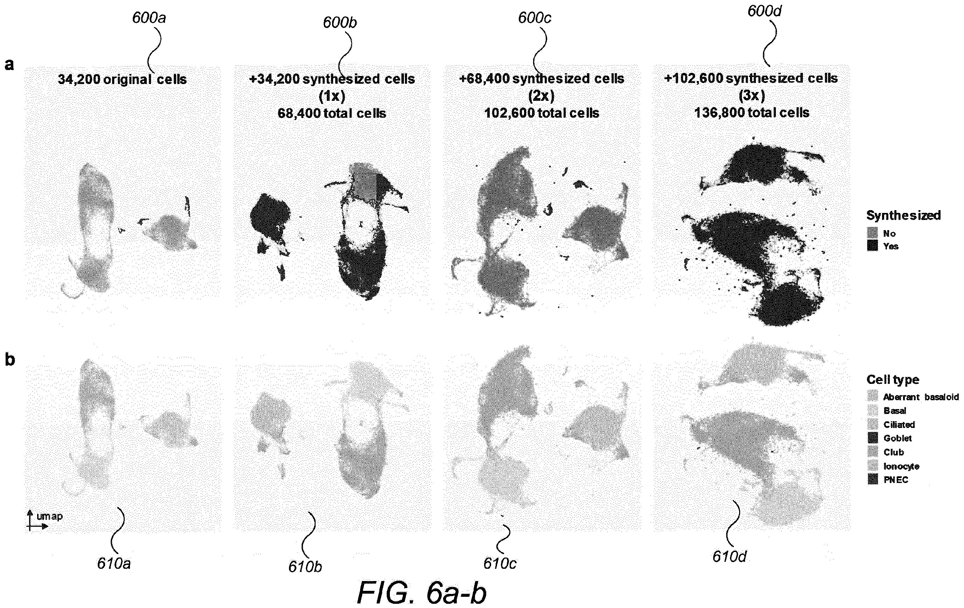

Figures 6a to 6e illustrate the performance of scGFT component 502 in augmenting experimental scRNA-seq datasets with synthesized gene expression profiles in which the resulting augmented dataset maintains distinct intrinsic cellular characteristics of the original experimental scRNA-seq dataset. In this example, the performance of scGFT component 502 is assessed for practical applications using experimental scRNA-seq data. The scRNA-seq dataset PRJEB44878 was used, which comprises 34,200 processed cells derived from primary small airway epithelial cells (SAECs) from both healthy individuals (n=3) and patients with chronic obstructive pulmonary disease (COPD) (n=3). These SAECs were subjected to *in-vitro* expansion and differentiation into pseudostratified epithelia via air-liquid interface (ALI) conditions. To model smoke-induced injuries in the small airways of healthy non-smokers and COPD smokers, the fully differentiated SAEC ALI cultures underwent exposure to either whole cigarette smoke over a period of four consecutive days or to ambient air serving as the control.

For this performance analysis of the scGFT component 502, multiple experiments were performed in which the scGFT component 502 synthesized new cells (e.g., new synthesized gene expression profiles) at varying expansion coefficients - single (1x), double (2x), and triple (3x) the number of original cells. As well, for each experiment, a different number of CCs that were modified was selected from 5, 10, 25, 50, 75, and 100 CCs. Using UMAP for a qualitative evaluation, the synthesized and real cells were projected onto the embedded manifold.

Figure 6a illustrates a UMAP visualization of the synthesized data with 100 CCs modified. This visualization demonstrates a substantial overlap across all expansion coefficients. Additionally, this finding indicates that the synthesis process did not introduce any non-biological artifacts. The UMAP visualities of Figure 6a is based on the PRJEB44878 dataset comprised of 34,200 processed single cells from primary small airway epithelial cells. The first column 600a shows the original dataset. Subsequent columns 600b-600d illustrate the original cells combined with cells synthesized by scGFT component 502 whilst modifying 100 complex components under different expansion coefficients: 1x synthesis resulting in 68,400 total cells (second column 600b), 2x synthesis resulting in 102,600 total cells (third column 600c), and 3x synthesis resulting in 136,800 total cells (fourth column 600d).

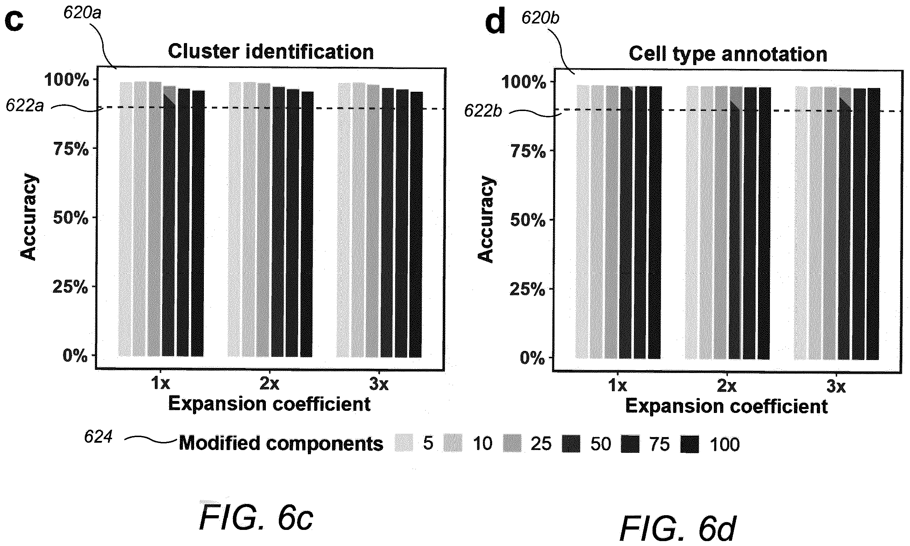

The evaluation was further quantitatively corroborated by a clustering analysis, in which both synthesized, and original, cells demonstrated an alignment exceeding 92% accuracy, with an average above 94% across all numbers of modified components and expansion coefficients tested as is shown in Figures 6b and 6c. It is noted that to process the experimental scRNA-seq data, the same analysis procedures both before and after synthesis were adhered to following the standard Seurat pipeline as previously discussed.

Genes with high variability manifest biological variations indicative of distinct cellular states. In order to further substantiate the preservation of inherent variability in the original cells within the synthesized data, the proportional overlap of the top 2,000 highly variable genes identified from the original and synthesized data across six different component modifications (5, 10, 25, 50, 75, and 100 components) and three expansion coefficients (1x, 2x, and 3x) was calculated. This assessment successfully demonstrated a high average overlap of $94 \pm 0.7\%$ (mean $\pm$ standard deviation) between two gene sets.

Cell type annotation is a crucial downstream analysis in single-cell RNA sequencing offering insights into the cellular heterogeneity within samples. The performance analysis was extended to evaluate the consistency of cell types in synthesized cells relative to the originals. To ensure an unbiased assessment, an algorithm called Sargent was used, which is an automated, cluster-free, score-based annotation method that classifies cell types based on distinct gene expression markers as outlined in Nouri, Nima and Gaglia, Giorgio and Kurlovs, Andre H and de Rinaldis, Emanuele and Savova, Virginia, 2023, "A marker gene-based method for identifying the cell-type of origin from single-cell RNA sequencing data." MethodsX (Elsevier) 10: 102196. Sargent is a transformation-free, cluster-free, single-cell annotation algorithm for rapidly identifying the cell types of origin based on cell type-specific markers. A comprehensive list of canonical epithelial subtype markers was curated by leveraging experts' knowledge and the literature. The Sargent algorithm identified distinct epithelial cell types, including goblet cells (n=15,472), ciliated cells (n=9,515), and basal cells (n=9,035), as well as less abundant subtypes such as club cells (n=94), aberrant basaloid cells (n=47), pulmonary neuroendocrine cells (PNECs) (n=21), and ionocytes (n=16). The UMAP visualizations also demonstrate a strong overlap between the synthesized gene expression profiles of synthesized cells that were output by the scGFT component 502 and the original gene expression profile datasets across all the expansion coefficients as illustrated in Figure 6b. Figure 6b illustrates another UMAP visualization of the synthesized data with 100 CCs modified. This is UMAP visualization of the same dataset as used in Figure 6a across the different expansion coefficients, and color-coded according to annotated cell types, which are in line with the cell types identified by the Sargent algorithm.

Figure 6c illustrates a bar plot 620a depicting the proportion of synthesized cells that accurately clustered with their corresponding original cells, shown for each expansion coefficient (x-axis). Each shaded color denotes the number of complex components modified as indicated in the "Modified" key 624, and the y-axis measures clustering accuracy. A horizontal dashed line 622a highlights the 90% accuracy threshold. This overlap was quantitatively supported by cell type annotation analysis illustrated in Figure 6d.

Figure 6d illustrates a bar plot 620b depicting the proportion of synthesized cells correctly annotated as identical to their corresponding original cell types, presented for each expansion coefficient (x-axis). Each shaded color indicates the number of complex components modified, and the y-axis measures annotation accuracy as indicated in the "Modified" key 624. A horizontal dashed line 622b indicates the 90% accuracy threshold. Both synthesized and original cells demonstrated concordance exceeding 95% accuracy in cell type labeling, with an average above 96% across all numbers of modified components from 5 to 100 and expansion coefficients.

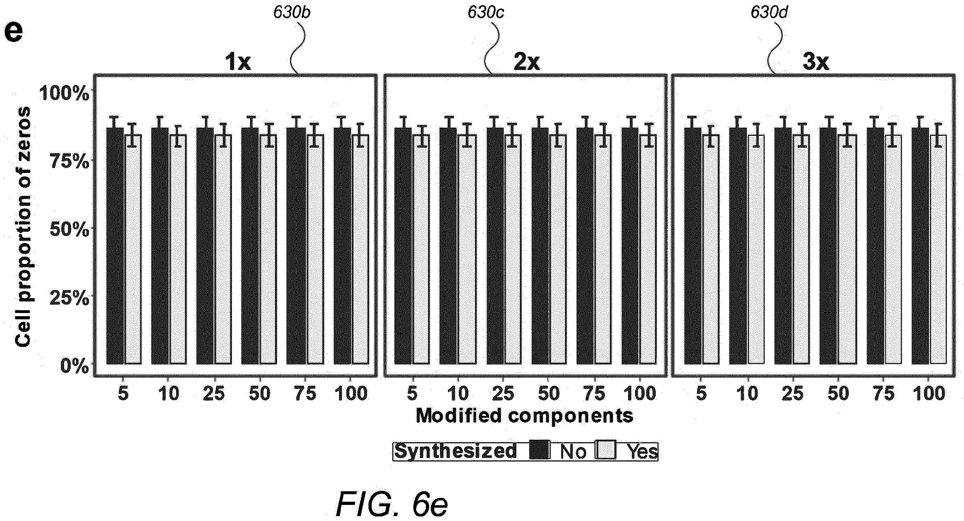

Figure 6e illustrates several bar plots 630b, 630c, and 630d corresponding to the 1x synthesis resulting in 68,400 total cells (e.g., second column 600b of Figure 6a), 2x synthesis resulting in 102,600 total cells (e.g., third column 600c of Figure 6a), and 3x synthesis resulting in 136,800 total cells (fourth column 600d of Figure 6a), respectively. These bar plots 630b-630d show cell data sparsity with the proportion of zeros calculated for original cells (dark bars) and synthesized cells (lightly shaded bars) across various the different numbers of complex components (e.g., 5, 10, 25, 50, 75 and 100) used for modifying the gene expression profiles along the (x-axis) for the different expansion coefficients (1x, 2x, and 3x, represented in bar plots 630b, 630c, and 630d, respectively). Error bars illustrate the mean $\pm$ standard deviation of the proportions calculated for each category.

Data sparsity, represented by zeros, is a characteristic feature of single-cell RNA sequencing datasets. This aspect of the synthesis process of scGFT component 502 is evaluated in Figure 6e where the number of zeros in synthesized and original cells is compared across various numbers of modified components and expansion coefficients.

This analysis reveals that the synthesized data exhibited a sparsity level slightly lower than the original data (85% versus 86%, respectively). The trend remained similar across all numbers of modified components and expansion coefficients, affirming that scGFT component 502 maintains the integrity of gene expression data without artificially denoising or extensively (< 1%) imputing.

The overall findings from experimental scRNA-seq datasets as illustrated in Figures 6a to 6e highlight the scGFT component's 502 capability to effectively augment scRNA-seq datasets in practical settings while preserving intrinsic cellular characteristics and biological plausibility.

These are both critical elements for robust downstream biotechnical/pharmacological analytical/processing applications.

In addition to preserving the intrinsic cellular characteristics and biological plausibility, the scGFT component 502 synthesizes distinct cell populations from individual cell gene expression profiles. Analyzing rare cell types in scRNA-seq data presents a formidable challenge due to their low abundances, significantly impacting statistical power and analytical precision. Augmenting these scRNA-seq datasets with scGFT component 502 addresses these issues with a higher biological plausibility and at a lower computational complexity compared with the capacity of generative models and/or manifolds when synthetically generating biologically plausible cellular populations solely from the gene expression profile of an individual cell.

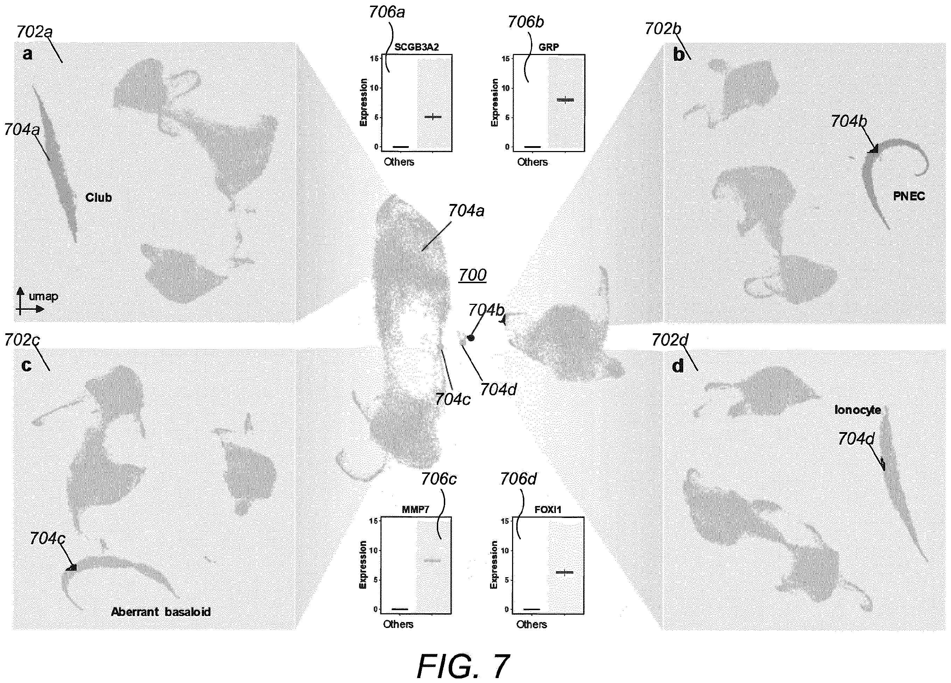

Figure 7 illustrates a UMAP representation 700 of the PRJEB44878 dataset with randomly selected individual rare cell types highlighted. The effectiveness of the scGFT component 502 in addressing the above challenge is tested by focusing on rare epithelial subtypes in the scRNA-seq PRJEB44878 experimental dataset, including $SCGB3A2^+$ club cells, $MMP7^+$ aberrant basaloid cells, $GRP^+$ PNECs, and $FOXI1^+$ ionocytes, each comprising less than 0.3% of the population. In this example, the scRNA-seq PRJEB44878 experimental dataset is augmented using the scGFT component 502 and is shown in the UMAP representation 700 to generate the biological characteristics of the rare epithelial subtypes in the scRNA-seq PRJEB44878 experimental dataset. Each of the rare epithelial cell subtypes are represented in UMAP 702a for club cells 704a, UMAP 702b for PNECs 704b, UMAP 702c for aberrant basaloid cells 704c, and UMAP 702d for ionocytes 704d, respectively. In this example, an individual cell from each population was randomly selected for analysis. Subsequently, 100 complex components were modified, and 5,000 synthesized cells (i.e., the synthesized gene expression profiles) were generated by scGFT component 502 for evaluating scGFT component's 502 ability to expand these rare cell profiles. Cells from other types are illustrated as light grey to distinguish the synthesized populations 704a-704d. This synthesis process effectively generated discrete cell populations, each remaining separate from others as illustrated by the UMAPs 700, 702a, 702b, 702c and 702d. The box plots 706a-706d also show the expression levels of the selected markers for each cell type. The upper and lower bounds of each of the boxplots 706a-706d represent the 75th and 25th percentiles, respectively. The center bars indicate the medians, and the whiskers denote values up to 1.5 interquartile ranges above the 75th or below the 25th percentiles. Quantitative support for this augmentation process came from annotation analyses of the integrated data, where both synthesized and original cells demonstrated a high degree of agreement in cell type labeling, with accuracy exceeding 98% on average across the four examined cell types.

This analysis shows the unique ability of scGFT component 502 to synthesize a distinctive population of cells exclusively based on the gene expression profile of an individual cell. This capability to expand rare cell types into scalable synthetic populations signifies a significant leap forward, surpassing the capabilities of current state-of-the-art generative methods.

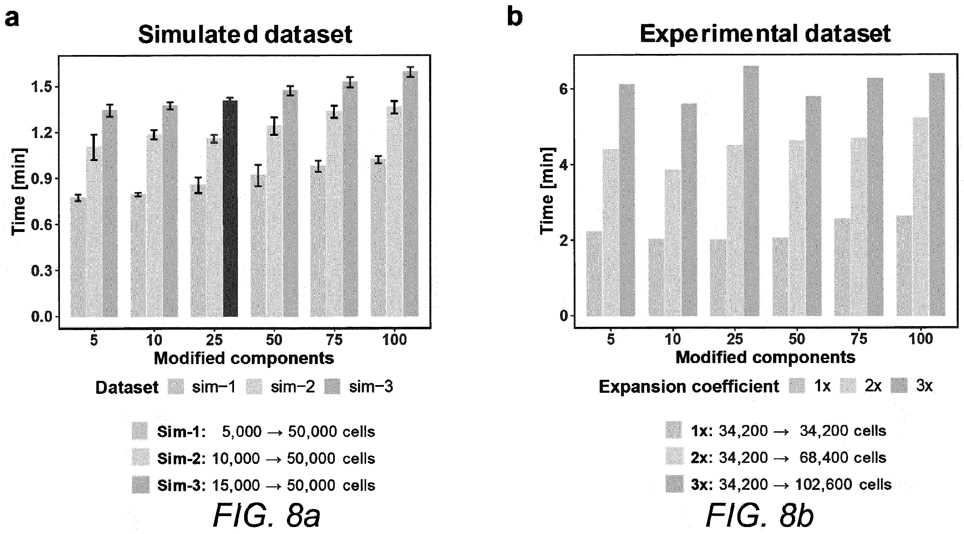

Figures 8a and 8b illustrate a bar plot 800a representing the computational efficiency of generating synthesized gene expression profiles from simulated gene expression profile dataset and a bar plot 800b representing the computational efficiency of generating synthesized gene expression profiles from experimental gene expression profile dataset. Computational efficiency is a critical and costly attribute for processing and analyzing gene expression datasets and the like, especially in research and development settings. The computational performance of the scGFT component 502 is evaluated by recording the time (e.g., runtime) it took to synthesize cells (e.g., synthesized gene expression profile datasets) from both simulated and experimental gene expression profile datasets. These tests were conducted on a computational device having a single-core setup with a 2.9 GHz processor and 64 GB of memory.

Referring to Figure 8a, in the analysis of simulated dataset, where the target number of synthesized cells was fixed at 50,000, it was observed that the synthesis time required by the scGFT component 502 varied based on the size of the original dataset and the number of modified CCs. Specifically, for the sim-1 dataset, which contained 5,000 cells, approximately 0.8 minutes were required to synthesize 50,000 cells when only five components were modified. This duration increased to approximately one minute when 100 components were modified. Similar patterns were observed for the larger sim-2 and sim-3 datasets containing 10,000 and 15,000 cells, with the longest runtime of approximately 1.5 minutes for maximum component modifications (n=100). The minimal increase in runtime in the scenario with a fixed number of CC modifications and a fixed number of synthesized cells is attributed to the DFT process applied to an increasing number of original cells.

Referring to Figure 8b, in the case of experimental data, the computational time was influenced by the number of cells to be synthesized and the number of components modified. The synthesis time ranged from approximately two minutes for a single expansion coefficient (1x; 34,200 cells) with minimal component modifications (n=5), to nearly six minutes for the highest expansion coefficient (3x; 102,600 cells) and maximum component modifications (n=100).

From both Figures 8a and 8b, it can be seen that the runtime of the scGFT component 502 when implemented scales additively with the number of original cells (o) and multiplicatively with both the number of synthesized cells ($\sigma$) and the number of modified CCs ($\kappa$). Consequently, the computational complexity can be represented as O(o+$\sigma\kappa$). A further advantage of the scGFT component 502 is its efficiency on, for example, uniprocessor systems without requiring graphical processing unit (GPU) acceleration, which the generative methods typically require.

Figures 5a to 8b have illustrated that the scGFT component 502, which is based on the synthetic gene expression data generation process(es) / component(s) 104, 110, 200, 400, and/or 410 of Figures 1a to 4b, is capable of generating realistic synthesized gene expression profiles that accurately capture the biological properties and/or mimic the transcriptional characteristics of the original real-world or experimental gene expression profiles of the original cells. This means that original gene expression profile datasets can be significantly augmented with synthesized gene expression profiles. This is particularly so for rare or smaller real-world gene expression profile datasets. As the adoption of artificial intelligence (AI) or machine learning (ML) based technologies continues to expand into the bioinformatics and pharmacological fields, there will be an increased demand for extensive large-scale datasets for effectively training the AI/ML models or classifiers for the myriad of downstream biotechnical/pharmacological applications and processes. The lack of extensive large-scale datasets is curtailing the predictive power of AI/ML models, particularly, in the single-cell transcriptomic realm where high-quality disease-specific training datasets are scarce. Thus, the scGFT component 502 as described herein, modifications thereof and the like has been shown to be capable of performing large-scale augmentation of real-world gene expression profile datasets by generating realistic synthetic single cell gene expression profiles, this directly addresses the issues of data scarcity.

The scGFT component 502 comprises a streamlined computational methodology grounded in mathematical rigor, leveraging the principles of the Fourier Transform. This rigor enables a successful in-silico synthesis process, carefully tailoring the original gene expression profile while preserving the variability of the original population. The performance of the scGFT component 502 has been validated in Figures 5a to 8b through comprehensive assessments using both simulated and experimental data across various scenarios. This validation illustrates scGFT component's 502 capability to generate synthetic gene expression profiles from synthetic cells that preserve the intrinsic characteristics delineated in the original scRNA-seq gene expression profile datasets, as evidenced by the above-mentioned evaluations, including clustering and cell-type annotation analyses. Furthermore, leveraging experimental data, the established features of scRNA-seq datasets were probed, confirming that scGFT component 502 generates dropout (zero) rates similar to those observed in real experimental gene expression profile datasets, while concurrently introducing batch variations akin to natural processing, ensuring the synthesis of realistic gene expression profiles.

As well, the scGFT component 502 facilitates the synthesis of an unlimited array of unique cells or synthesized gene expression profiles from experimentally observed single-cell gene expression profiles. Notably, the synthesis process can initiate from just a single cell, surpassing the capabilities of current state-of-the-art generative models. Furthermore, the scGFT component 502 obviates the need for manifold reduction techniques, extensive training phases, and complex hyperparameter tuning strategies common in conventional generative models. These features ensure scGFT component 502 can efficiently execute without requiring high-performance GPU resources, making it accessible across various computing devices, systems, and settings.

The scGFT component 502 also enables controlled deviation from original single cell gene expression profiles through allowing a user to modify the number of CCs to meet a desired deviation of the synthesized gene expression profile dataset from the original gene expression profile dataset. An excessive increase in the number of modified components amplifies the profile disparity between synthesized cells or synthesized gene expression profiles and their originals.

As discussed, or described herein, the scGFT component 502 can be used to generate an augmented real-world gene expression profile dataset that includes the original or real-world gene expression profile dataset used to generate a synthetic gene expression dataset and the resulting synthetic gene expression dataset. The scGFT component 502 and the resulting augmented real-world gene expression profile dataset has technical character, and contributes to producing a technical effect that serves a technical purpose, by the application of the augmented real-world gene expression profile dataset to the bioinformatics and/or pharmacological fields of technology, especially in cases where there is a scarcity of samples in a real-world gene expression profile dataset. Once the scGFT component 502 has generated an augmented real-world gene expression profile dataset, the augmented real-world gene expression profile dataset may be used and/or adapted for use in a variety of specific technical bioinformatics/pharmacological applications and implementations thereof. For example, some specific technical bioinformatics/pharmacological applications and implementations include sending, ingesting or applying the augmented real-world gene expression profile dataset in, for example, a downstream bioinformatics/pharmacological pipeline 106 of Figure 1a for use in technical applications such as, without limitation, for example drug discovery; therapeutics; novel gene discovery and applications thereof; disease detection, treatment and/or prevention; analyzing tissue and cell variability and biology, and endotype, phenotype and biomarker identification; training classifiers used in clinical settings including, without limitation, for example patient and disease stratification and endotyping; further downstream analysis including, without limitation, for example MOA and the like; toxicity and/or efficacy prediction/classification of cellular samples from wet lab analysis or high throughput screening during drug discovery/optimization and testing on said cellular samples; training and fine tuning of biological and/or genomic based foundational models the results of which are applied in subsequent in-vivo trials and/or in-vitro wet lab analysis and other technical biological/pharmacological analyses/processes; and/or for use in subsequent in-vivo trials and/or in-vitro wet lab analysis; and other technical biological/pharmacological analyses/processes and the like.

The scGFT component 502 is capable of supporting the development of newly emerged AI/ML based technologies, such as foundational models for scRNA-seq, particularly in scenarios where fine-tuning is challenging due to scarce sample availability of the variety of gene expression profile datasets used to train such foundational models. As well, by facilitating the generation of anonymized, disease-specific synthetic patient repositories, scGFT component 502 is able to address the financial and ethical constraints associated with clinical settings while supporting the development of robustly trained AI/ML-based classifiers essential for the design of clinical trials and/or drug discovery/optimization. Thus, scGFT component 502 serves as an ethically sound and cost-effective catalyst for progress in therapeutic and drug discovery/optimization. Given that the scGFT component 502 can be implemented based on the Seurat framework, it is clear to the skilled person in bioinformatics that the scGFT component 502 and/or methodology has many technical bioinformatic/-pharmacological applications that enables its integration into existing bioinformatics/pharmacological pipelines.

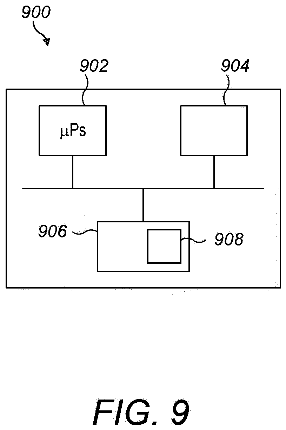

Figure 9 is a schematic illustration of a system/apparatus for performing methods described herein. The system/apparatus shown is an example of a computing device. It will be appreciated by the skilled person that other types of computing devices/systems may alternatively be used to implement the methods described herein, such as a distributed computing system.

The apparatus (or system) 900 comprises one or more processors 902. The one or more processors control operation of other components of the system/apparatus 900. The one or more processors 902 may, for example, comprise a general-purpose processor. The one or more processors 902 may be a single core device or a multiple core device. The one or more processors 902 may comprise a central processing unit (CPU) or a graphical processing unit (GPU). Alternatively, the one or more processors 902 may comprise specialised processing hardware, for instance a RISC processor or programmable hardware with embedded firmware. Multiple processors may be included.

The system/apparatus comprises a working or volatile memory 904. The one or more processors may access the volatile memory 904 in order to process data and may control the storage of data in memory. The volatile memory 904 may comprise RAM of any type, for example Static RAM (SRAM), Dynamic RAM (DRAM), or it may comprise Flash memory, such as an SD-Card.

The system/apparatus comprises a non-volatile memory 906. The non-volatile memory 906 stores a set of operation instructions 908 for controlling the operation of the processors 902 in the form of computer readable instructions or code. The non-volatile memory 906 may be a memory of any kind such as a Read Only Memory (ROM), a Flash memory, or a magnetic drive memory.

The one or more processors 902 are configured to execute operating instructions 908 to cause the system/-apparatus to perform any of the methods or processes described herein with reference to Figures 1a to 8b. The operating instructions 908 may also comprise instructions or code (i.e., drivers) relating to the hardware components of the system/apparatus 900, as well as instructions or code relating to the basic operation of the system/apparatus 900. Generally speaking, the one or more processors 902 execute one or more instructions or code of the operating instructions 908, which are stored permanently or semi-permanently in the non-volatile memory 906, using the volatile memory 904 to temporarily store data generated during execution of said operating instructions 908.

Implementations of the methods and/or processes described herein may be realised as in digital electronic circuitry, integrated circuitry, specially designed ASICs (application specific integrated circuits), computer hardware, firmware, software, and/or combinations thereof. These may include computer program products (such as software stored on e.g., magnetic discs, optical disks, memory, Programmable Logic Devices, in cloud storage, an app store and the like) comprising computer readable instructions or code that, when executed by a computer, such as that described in relation to Figure 9, cause the computer to perform one or more of the methods described herein.

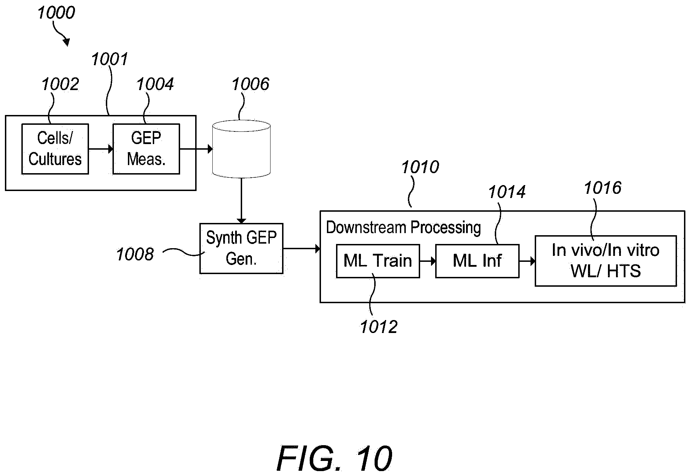

Figure 10 is a schematic illustration of a computing system pipeline 1000 configured for performing methods and/or processes as described herein. The computing system pipeline 1000 may further modify the computing system/-apparatus 900 of Figure 9 by including various further biological/pharmacological systems, computing systems, subsystems, components, devices, units and/or modules each of which may implement one or more aspects of the methods, processes, systems and/or apparatus as described with reference to Figures 1a to 9. It will be appreciated by the skilled person that other types of biological/pharmacological systems, computing systems, subsystems, components, devices, units and/or modules may alternatively be used in the computing system pipeline 1000 to implement the methods, processes, systems and/or apparatus 1a to 9 as described herein.

The computing system pipeline 1000 includes a gene expression profiling platform 1001 configured for generating real-world gene expression profile datasets. The gene expression profiling platform 1001 may include, without limitation, for example a cell/culture platform 1002 configured for producing/outputting cellular samples from one or more subjects/cultures according to the requirements of an operator/user (e.g., testing subjects, cell sample testing, cell experiments, growing cell lines, assays, and compound/drug analyses on cell lines/cell samples etc., and the like). The gene expression profiling platform 1001 may also include a gene expression profile measurement unit 1004 configured for measuring real-world gene expression profiles associated with one or more cells in relation to one or more subjects or cultures from the produced cellular samples. The gene expression profile measurement unit 1004 may perform any type of gene expression profiling such as, without limitation, for example scRNA-seq sequencing or microarrays for quantifying a set of predetermined sequences, and/or any other type of gene expression profiling. The generated real-world gene expression profile datasets are stored in gene expression profile dataset storage unit 1006. The gene expression profile dataset storage unit 1006 is configured for collecting and storing the measured real-world gene expression profiles in one or more gene expression profile datasets according to one or more biological and/or pharmacological criteria and the like. The real-world gene expression profile datasets may then be used to generate synthetic gene expression profile datasets using a synthetic gene expression profile generation unit 1008 that is configured to implement the synthetic gene expression data generation process(es) / component(s) 104, 110, 200, 400, and/or 410 described with reference to Figures 1a to 4b, and/or scGFT component 502 as described with reference to Figures 5a to 8b, combinations thereof, modifications thereto, and/or as the application demands. The synthetic gene expression profile generation unit 1008 is configured for receiving a selected gene expression profile dataset from the gene expression profile dataset storage unit 1006 and generating a synthetic gene expression profile dataset based on the selected real-world gene expression profile dataset as described herein. The synthetic gene expression profile generation unit 1008 outputs a generated synthetic gene expressions profile dataset for use in biological/pharmacological downstream analysis/processing. In this example, a downstream processing unit 1010 may be configured for receiving and processing the generated synthetic gene expression profile dataset and/or the corresponding selected real-world gene expression profile dataset in relation to a technical biological and/or pharmacological objective or application.

For example, the technical biological and/or pharmacological objective or application may be based on the technical biological and/or pharmacological objectives or applications as described with reference to Figures 1a to 3c and/or Figures 4a to 8b. Although some examples technical biological and/or pharmacological objectives or applications are described herein, this by way of example only and downstream processes/analysis is not so limited, it is to be appreciated by the skilled person that the synthetic gene expression profile datasets that are generated may be used for many or a plethora of technical biological/pharmacological applications and the like.

In this example, the downstream processing unit 1010 may be configured to include an ML training module 1012 configured for generating a training dataset from a selected received synthetic gene expression profile dataset generated from synthetic gene expression profile generation unit 1008 and/or from the corresponding real-world gene expression profile dataset used for generating the synthetic gene expression profile dataset. For example, the ML training module 1012 may use the training dataset and an ML algorithm for training model parameters for an ML model for predicting or classifying a technical biological objective and/or technical pharmacological objective in relation to the synthetic gene expression profile dataset and/or the corresponding selected real-world gene expression profile dataset. In another example, the ML training module 1012 may use the training dataset and an ML algorithm for training model parameters for an ML model as described with reference to Figures 3a to 3c and/or as herein described with reference to any of Figures 1a to 8b. Although several ML models for technical applications have been described herein, this is by way of example only and the technical applications are not limited to only the ML models as described herein, it is to be appreciated by the skilled person in the genomic/biological/pharmacological fields that the synthetic gene expression profile dataset that are generated from a real-world gene expression profile dataset and which accurately capture the biological properties and/or mimic the transcriptional characteristics of the original real-world gene expression profile dataset may be used for many other technical biological and/or pharmacological applications and the like. Once the ML training module 1012 has successfully trained the model parameters of an ML model, the resulting ML model may be used by an ML inferencing module 1014 configured for performing inferencing using the trained ML model for, without limitation, for example predicting or classifying the biological objective and/or pharmacological objective in relation to input data representative of at least a real-world gene expression profile or a synthetic gene expression profile and the like. The ML inferencing module 1014 may use the trained ML model for, without limitation, for example inferencing, prediction and/or classification and the like as described with reference to any Figures 3a to 3c and/or as herein described with reference to any of Figures 1a to 8b. Although several technical applications using ML inferencing have been described herein, this is by way of example only and the technical applications are not limited to only the ML models / inferencing as described herein, it is to be appreciated by the skilled person in the genomic/biological/pharmacological fields that, given the synthetic gene expression profile dataset generated from a real-world gene expression profile dataset as herein described accurately captures the biological properties and/or mimic the transcriptional characteristics of the original real-world gene expression profile dataset, then the skilled person would understand that the generated synthetic gene expression profile dataset can be applied and is significantly useful many technical biological/pharmacological applications. The downstream processing unit 1010 may further include an output results module 1016 configured to output data representative of any results (e.g., short list of candidate drugs/compounds, patient stratification results etc.) resulting from a technical application of the predicted and/or classified biological objective and/or pharmacological objective. The output results module 1016 may send the output data representative of any results for further downstream processing/testing such as, without limitation, for example *in vivo* trials, *in vitro* wet lab analysis, and/or high-throughput screening laboratory analysis, and/or any other technical biological/pharmacological processing/testing and/or experiments as the application demands.

Any system feature as described herein may also be provided as a method feature, and vice versa. As used herein, means plus function features may be expressed alternatively in terms of their corresponding structure. In particular, method aspects may be applied to system aspects, and vice versa.

Furthermore, any, some and/or all features in one aspect can be applied to any, some and/or all features in any other aspect, in any appropriate combination. It should also be appreciated that particular combinations of the various features described and defined in any aspects of the invention can be implemented and/or supplied and/or used independently.

Although several embodiments have been shown and described, it would be appreciated by those skilled in the art that changes may be made in these embodiments without departing from the principles of this disclosure, the scope of which is defined in the claims.

## Claims

1. A computer-implemented method for generating synthetic gene expression profile datasets for downstream biological/pharmacological analysis, processing and/or applications, the method comprising:

   receiving a gene expression profile dataset comprising data representative of at least one gene expression profile;
   computing a complex gene expression profile dataset based on applying a discrete Fourier transform to data representative of each gene expression profile of the received gene expression profile dataset;
   sampling the complex gene expression profile dataset based on using a statistical distribution to sample and modify a predetermined number of one or more complex components of each complex gene expression profile for generating a plurality of synthetic complex gene expression profiles;
   computing a real-valued synthetic gene expression profile dataset based on applying an inverse discrete Fourier transform to each of the plurality of synthetic complex gene expression profiles; and
   outputting data representative of the real-valued synthetic gene expression profile dataset for downstream biological/pharmacological analysis, processing and/or applications.

2. The computer-implemented method of claim 1, wherein receiving the gene expression profile dataset further comprises receiving a gene expression profile dataset derived from real-world measurements of gene expression from one or more cells of at least one subject.

3. The computer-implemented method of claims 1 or 2, wherein the downstream biological/pharmacological analysis, processing and/or applications comprise one or more from the group of:

   drug discovery or optimization;
   therapeutics;
   novel gene discovery;
   patient stratification;
   endotyping;
   disease detection/prevention;
   *in vivo* trials, *in vitro* wet lab analysis, and/or high-throughput screening laboratory analysis;
   any other type of biological/pharmacological analysis, processing and/or applications using at least the output real-valued synthetic gene expression profile dataset and, optionally, the corresponding real-world gene expression profile dataset; and
   any other type of technical biological/pharmacological processing or applications using at least the output real-valued synthetic gene expression profile dataset and, optionally, the corresponding real-world gene expression profile dataset.

4. The computer-implemented method of any preceding claim, wherein:

   each gene expression profile is represented as a vector of length $N$, where $N > 0$ is the total number of genes and each element, n, of the vector represents a gene index of the gene expression profile for $0 \leq n \leq (N-1)$; and

each complex gene expression profile is represented as a vector of length *N*, where *N* is the total number of genes and each *k*-th element of the vector represents a complex component comprising a complex value with an amplitude and phase, for $0 \leq k \leq (N-1)$.

5. The computer-implemented method of any preceding claim, wherein the gene expression profile dataset comprises data representative of gene expression profiles based on one from the group of:

cell gene expression profiles;
single-cell Ribonucleic acid, RNA, sequences; or
any other particular type of gene expression profile associated with a subject or cellular structure.

6. The computer-implemented method of any preceding claim, said sampling the complex gene expression profile dataset for generating a plurality of synthetic complex gene expression profiles further comprising:

computing the mean and variance for each complex component of each complex gene expression profile of the complex gene expression profile dataset;
selecting a statistical distribution having said mean and variance for each complex component of the complex gene expression profile dataset;
iteratively performing, until a predetermined number of synthetic complex gene expression profiles have been generated, the following steps of:

generating, by sampling the statistical distribution for each complex component, sampled modification factors corresponding to the N complex components of each complex gene expression profile;
generating synthetic complex gene expression profiles based on applying, to each complex gene expression profile, a predetermined number of randomly selected sampled modification factors to corresponding complex components and their conjugate pairs of said each complex gene expression profile; and
updating the synthetic complex gene expression profile dataset with the generated synthetic complex gene expression profiles.

7. The computer-implemented method of claim 6, wherein:

when the length of the gene expression profile, *N*, is an even number, then the complex conjugate pair of the (N/2)-th complex component is itself and the remaining complex conjugate pair of a *k-th* complex component, for $1 \leq k < N12$, is the *(N-k)-th* complex component; and
when the length of the gene expression profile, *N*, is an odd number, then the complex conjugate pair of a *k-th* complex component, for $1 \leq k < (N-1)/2$, is the *(N-k)-th* complex component; and, as an option,
wherein the component of each complex gene expression profile corresponding to the Direct Current component is excluded from being randomly selected when applying sample modification factors.

8. The computer-implemented method of any preceding claim, further comprising:

determining one or more cell groups in the received gene expression profile of the complex gene expression profile dataset in which each cell group has a similar or the same gene expression profile; and
wherein said sampling the generated complex gene expression profiles for generating a plurality of synthetic complex gene expression profiles is performed independently for each determined one or more cell groups.

9. The computer-implemented method of any of claim 8, wherein:

when the number of cells in each cell group is greater than 1, then the selected statistical distribution used to generate each sample modification factor of each complex component uses a mean of 2 and the computed variance for said complex component for that cell group; and
when the number of cells in each cell group is 1, then the selected statistical distribution used to generate each sample modification factor of each complex component uses a mean of 2 and a variance of 1.

10. The computer-implemented method of any preceding claim, wherein the statistical distribution comprises a probability distribution based on a Gaussian or Normal distribution.

11. The computer-implemented method of any preceding claim, wherein:

said outputting data representative of the real-value synthetic gene expression profile dataset for downstream biological/pharmacological analysis, processing and/or applications further comprising:

generating an augmented gene expression profile dataset based on the real-value synthetic gene expression profile dataset and the real-world gene expression profile dataset;

applying the augmented gene expression profile dataset to a foundational model for training or fine tuning said foundational model, the foundational model representing a biological or cellular process;

using the foundational model to model a desired biological or cellular process the results of which are used in biological/pharmacological downstream analysis and/or processes; or

said outputting data representative of the real-valued synthetic gene expression profile dataset for downstream biological/pharmacological analysis, processing and/or applications further comprising:

generating a training dataset from the real-valued synthetic gene expression profile dataset, wherein the training dataset includes the real-valued synthetic gene expression profile dataset and at least the corresponding real-world gene expression profiles from the real-world gene expression profile dataset used to generate the real-valued synthetic gene expression profile dataset;

iteratively training, using a machine learning algorithm, model parameters of a machine learning, ML, model until a stopping criterion is reached, the ML model configured for predicting / classifying a biological objective and/or pharmacological objective based on the gene expression profiles of the training dataset;

performing ML inference by inputting at least data representative of a real-world gene expression profile associated with a subject or data representative of a theoretical gene expression profile to the trained ML model, wherein the trained ML model processes the input real-world gene expression profile or theoretical gene expression profile and outputs a prediction / classification associated with the biological objective and/or the pharmacological objective in relation to the gene expression profiles of the training dataset;

wherein predicting / classifying the biological objective corresponds to at least one of predicting / classifying cell-type, disease, disease associated with cell-type, and/or any other biological objective; and/or

predicting / classifying the pharmacological objective corresponds to at least one of predicting / classifying drug efficacy, drug toxicity, drug and/or drug dosage based on a detected disease, and/or any other pharmacological objective.

12. The computer-implemented method of any preceding claim, wherein the real-world gene expression profile dataset is associated with a particular disease, the particular disease comprising a plurality of different disease endotypes, the method further comprising:

prior to generating the real-value synthetic gene expression profile dataset, dividing the real-world gene expression profile dataset into a plurality of subsets of the real-world gene expression profile dataset, each real-world gene expression profile sub-dataset associated with a different disease endotype of the particular disease; and

generating, for each real-world gene expression profile sub-dataset, a corresponding synthesized gene expression profile sub-dataset in accordance with the method of claim 1, wherein the downstream biological/pharmacological analysis, processing and/or applications further comprising:

generating augmented gene expression profile sub-datasets based on storing each real-value synthetic gene expression profile sub-dataset with the corresponding synthetic gene expression profile sub-dataset;

generating a training dataset based on labelling and including each gene expression profile data instance of each augmented gene expression profile sub-dataset according to the corresponding disease endotype;

applying the training dataset to a patient / disease stratification machine learning, ML, model for classifying an input gene expression profile of a subject as being associated with one of the disease endotypes of the plurality of different disease endotypes; and

performing patient / disease stratification based on applying a plurality of gene expression profiles from a cohort of patients to the trained patient / disease stratification ML model.

13. The computer-implemented method of any preceding claim, wherein:

said computing the complex gene expression profile dataset further comprising:

pre-processing the received gene expression profile dataset to form a normalised gene expression profile dataset; and

computing the complex gene expression profile dataset based on applying a discrete Fourier transform to data representative of each normalised gene expression profile of the normalized gene expression profile dataset; and

said computing the real-valued synthetic gene expression profile dataset further comprising:

applying the inverse discrete Fourier transform to each of the plurality of synthetic complex gene expression profiles to form a normalised synthetic gene expression profile dataset; and

post-processing the normalised synthetic gene expression profile dataset based on back translating or reverse-normalising the normalised synthetic gene expression profile dataset into the real-valued synthetic gene expression profile dataset associated with the received gene expression profile dataset.

14. An apparatus or computing system comprising a processor, a memory unit and a communication interface, wherein the processor is connected to the memory unit and the communication interface, wherein the processor and memory are configured to implement the computer-implemented method according to any of the preceding claims.

15. A computer program product comprising data or instruction code, which when executed on a processor, causes the processor to implement the computer-implemented method of any of claims 1 to 13.

100

Real-world gene expression profile dataset ~ 102

Generation of Synthetic Gene Expression Profiles

~ 104

Downstream Bioinformatics/Pharmacological
Processing
- Drug discovery
- Therapeutics
- Novel gene discovery
- Disease detection/prevention
- In-vivo trials and/or in-vitro Wet-lab analysis

~ 106

# FIG. 1A

110

```
┌──────────────────────────────────────┐
│   Receiving a gene expression profile dataset   │──112
└──────────────────────────────────────┘
                    │
                    ▼
┌──────────────────────────────────────┐
│  Applying a Discrete Fourier Transform to data   │──114
│  representative of the received gene expression  │
│     profile dataset to form complex gene         │
│           expression data                        │
└──────────────────────────────────────┘
                    │
                    ▼
┌──────────────────────────────────────┐
│  Sampling the complex gene expression data to    │──116
│  generate a plurality of samples of synthetic    │
│        complex gene expression data              │
└──────────────────────────────────────┘
                    │
                    ▼
┌──────────────────────────────────────┐
│ Applying an Inverse Discrete Fourier Transform   │──118
│ to the plurality of samples of synthetic complex │
│  gene expression data to form a real-valued      │
│   synthetic gene expression profile dataset      │
└──────────────────────────────────────┘
                    │
                    ▼
┌──────────────────────────────────────┐
│   Outputting the real-valued synthetic gene      │──120
│  expression profile dataset for downstream       │
│              processing                          │
└──────────────────────────────────────┘
```

*FIG. 1B*

*200*

Receive gene expression profile dataset — 202

*204*

Generation of Synthetic Gene Expression Profiles

Applying Discrete Fourier Transform to received gene expression profile dataset to form a complex gene expression profile dataset — 206

Compute statistical distribution for each complex component — 208

Generate sample modification factors for the complex components using the statistical distribution — 210

Generating synthetic complex gene expression profiles based on randomly selecting a predetermined set of sample modification factors and applying to corresponding components of each complex gene expression profile — 212

Updating a synthetic complex gene expression profile dataset with the generated synthetic complex gene expression profiles — 214

Generate further synthetic complex gene expression profiles

More synthetic complex gene expression profiles required? — 216

*218*

Applying Inverse Discrete Fourier Transform to the set of synthetic complex gene expression profiles to form a set of real-valued synthetic gene expression profiles — 220

222 — Outputting the set of real-valued synthetic gene expression dataset for downstream processing/analysis

## FIG. 2

300

Training dataset based on synthetic gene expression dataset, and, as an option, corresponding real-world gene expression data ~301

ML Model Training ~302

Iteratively training ML model based on training dataset in relation to biological/ pharmacological/therapeutic/drug discovery or optimisation applications ~303

Output ML Model for inference in relation to biological/pharmacological/therapeutic/drug discovery or optimisation applications ~304

# FIG. 3a

*310*

Receive a real-world gene expression profile dataset associated with a particular disease ~311

Analyse and split the received real-world gene expression profile dataset into real-world gene expression profile sub-datasets related to disease endotypes of the particular disease ~312

Generate a synthesized gene expression profile dataset for each real-world gene expression profile sub-dataset ~313

Generate augmented gene expression profile datasets for each disease endotype ~314

Generate a training dataset by labelling each gene expression profile data instance in each augmented gene expression profile datasets with at least the corresponding disease endotype ~315

Train an AI/ML model to classify a gene expression profile of a subject as being associated with one of the plurality of disease endotypes ~316

Performing patient / disease stratification based on applying a plurality of gene expression profiles of patients to the trained AI/ML model for classifying each patient as being associated with one of the plurality of disease endotypes ~317

Outputting patient and/or disease stratification data based at least on the classifications ~318

## FIG. 3b

*320*

Receiving a real-world gene expression profile dataset for a particular disease associated with a one or more drug/compounds having known toxicity or efficacy — *321*

Analyse and split the received real-world gene expression profile dataset into real-world gene expression profile sub-datasets related to each drug/ compound and associated toxicity or efficacy — *322*

Generate a synthesized gene expression profile dataset for each real-world gene expression profile sub-dataset — *323*

Generate augmented gene expression profile datasets for each drug/ compound and associated toxicity or efficacy — *324*

Generate a training dataset by labelling each gene expression profile data instance in each augmented gene expression profile datasets with at least the corresponding drug/compound, toxicity or efficacy — *325*

Train an AI/ML model to predict/classify the toxicity or efficacy of a drug/ compound candidate based on an input gene expression profile of a cellular sample affected by the drug candidate — *326*

Performing wet lab analysis or high throughput screening on cellular samples of a plurality of the cellular samples affected by a group of drug/ compound candidates — *327*

Performing scRNA-seq on the cellular samples to generate real-world gene expression profiles for each of the cellular samples — *328*

Generating a shortlist of drug/compound candidates for the particular disease based on applying the real-world gene expression profiles to the trained AI/ML model for predicting/classifying the toxicity or efficacy of each drug/compound candidate — *329*

Outputting a shortlist of drug/compound candidates for the particular disease having desired toxicity or desired efficacy for use in in-vivo trials, further in-vitro wet-lab analysis, and/or high throughput screening — *330*

*FIG. 3c*

*FIG. 4a*

G(Count) ——————————————————————→ SG(Count)'

$\downarrow$ 422

Norm     **Fourier transform based generative model**     Count Adjust

424

438

NGData → DFT → Modification → IFT → Smooth & ReLU → NSG(Data)'

426     428     430     432     434/ 435     436

420    440

*FIG. 4b*

**FIG. 5a**

**FIG. 5b**

**FIG. 5c**

FIG. 6a-b

**FIG. 6c**

**FIG. 6d**

*FIG. 6e*

FIG. 7

EP 4 668 278 A1

*FIG. 8b*

*FIG. 8a*

*900*

*902*  *904*

μPs

*906*  *908*

FIG. 9

FIG. 10

# EUROPEAN SEARCH REPORT

**Application Number**

EP 24 30 5992

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ANAYA-ISAZA ANDRÉS ET AL: "Fourier transform-based data augmentation in deep learning for diabetic foot thermograph classification", BIOCYBERNETICS AND BIOMEDICAL ENGINEERING, vol. 42, no. 2, 1 April 2022 (2022-04-01), pages 437-452, XP093224523, PL ISSN: 0208-5216, DOI: 10.1016/j.bbe.2022.03.001 Retrieved from the Internet: URL:https://pdf.sciencedirectassets.com/287018/1-s2.0-S0208521622X00028/1-s2.0-S0208521622000171/main.pdf?X-Amz-Security-Token=IQoJb3JpZ21uX2VjEHgaCXVzLWVhc3QtMSJHMEUCI F5jnVBD6UhDbgZxSurPigTfwg01o6qBY8ejs4MDB4k HAiEAiYwWOEZAh3hmveV5s+RHeMzgVMPdD3ZndHE67 2/MGs0qswUIERAFGgwwNTkwMDM1NDY4NjUiDH7vQR3 78xAx/HdmW> * abstract; figure 2 * | 1-15 | INV. G16B25/10 G16B40/20 |
| A | ----- KR 2021 0123824 A (UNIV INHA RES & BUSINESS FOUND [KR]) 14 October 2021 (2021-10-14) * claims 1-8 * | 1-15 | TECHNICAL FIELDS SEARCHED (IPC) G16B |
| T | ----- Nouri Nima: "scGFT: single-cell RNA-seq data augmentation using generative Fourier transformer", bioRxiv, 13 July 2024 (2024-07-13), XP093225009, DOI: 10.1101/2024.07.09.602768 Retrieved from the Internet: URL:https://www.biorxiv.org/content/10.1101/2024.07.09.602768v1.full.pdf * the whole document * ----- | | |

The present search report has been drawn up for all claims

| Place of search | Date of completion of the search | Examiner |
|---|---|---|
| Munich | 18 November 2024 | Heidrich, Alexander |

EPO FORM 1503 03.82 (P4C01)

## ANNEX TO THE EUROPEAN SEARCH REPORT
## ON EUROPEAN PATENT APPLICATION NO.

EP 24 30 5992

This annex lists the patent family members relating to the patent documents cited in the above-mentioned European search report.
The members are as contained in the European Patent Office EDP file on
The European Patent Office is in no way liable for these particulars which are merely given for the purpose of information.

18-11-2024

| Patent document cited in search report | Publication date | Patent family member(s) | Publication date |
|---|---|---|---|
| KR 20210123824 A | 14-10-2021 | NONE | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Scaling up single-cell RNA-seq data analysis with CellBridge workflow.. **NOURI, NIMA** ; **ANDRE H KURLOVS** ; **GIORGIO GAGLIA** ; **EMANUELE DE RINALDIS** ; **VIRGINIA SAVOVA**. Bioinformatics. Oxford University Press, 2023, vol. 39, 760
- Dictionary learning for integrative, multimodal and scalable single-cell analysis.. **HAO, YUHAN** ; **STUART, TIM** ; **KOWALSKI, MADELINE H** ; **CHOUDHARY, SAKET** ; **HOFFMAN, PAUL** ; **HARTMAN, AUSTIN** ; **SRIVASTAVA, AVI** ; **MOLLA, GESMIRA** ; **MADAD, SHAISTA** ; **FERNANDEZ-GRANDA, CARLOS**. Nature biotechnology. Nature Publishing Group, 2024, vol. 42, 293-304
- Fast, sensitive and accurate integration of single-cell data with Harmony.. **KORSUNSKY, ILYA** ; **MILLARD, NGHIA** ; **FAN, JEAN** ; **SLOWIKOWSKI, KAMIL** ; **ZHANG, FAN** ; **WEI, KEVIN** ; **BAGLAENKO, YURIY** ; **BRENNER, MICHAEL** ; **LOH, PO-RU** ; **RAYCHAUDHURI, SOUMYA**. Nature methods. Nature Publishing Group, 2019, vol. 16, 1289-1296
- A marker gene-based method for identifying the cell-type of origin from single-cell RNA sequencing data.. **NOURI, NIMA** ; **GAGLIA, GIORGIO** ; **KURLOVS, ANDRE H** ; **DE RINALDIS, EMANUELE** ; **SAVOVA, VIRGINIA**. MethodsX. Elsevier, 2023, vol. 10, 102196